# EUROPÄISCHE PATENTSCHRIFT

(11) **EP 1 516 054 B1**
(45) Veröffentlichungstag und Bekanntmachung des Hinweises auf die Patenterteilung: **02.12.2009**
(21) Anmeldenummer: 02806793.2
(22) Anmeldetag: 21.08.2002
(51) Int. Cl.: C12N 15/62, C07K 19/00, C12N 5/10, A61K 38/17

(54) **FUSIONKONSTRUKTE, ENTHALTEND AKTIVE ABSCHNITTE VON TNF-LIGANDEN**
FUSION CONSTRUCTS CONTAINING ACTIVE SECTIONS OF TNF LIGANDS
STRUCTURES DE FUSION CONTENANT DES PARTIES ACTIVES DE LIGANDS TNF

(30) Priorität: 10.02.2002 DE 10205368; 11.02.2002 DE 10205583
(43) Veröffentlichungstag der Anmeldung: 23.03.2005
(73) Patentinhaber: Topotarget Switzerland SA, 1004 Lausanne (CH)
(72) Erfinder: GAIDE, Oliver, CH-1006 Lausanne (CH); SCHNEIDER, Pascal, Ch-1066 Epalinges (CH); TSCHOPP, Jürg, CH-1066 Epalinges (CH)
(74) Vertreter: Tetaz, Franck Claude Edouard
(86) Internationale Anmeldenummer: PCT/EP2002/009354
(87) Internationale Veröffentlichungsnummer: WO 2003/068977

(56) Entgegenhaltungen:
- WO-A-01/49866
- WO-A2-01/83525
- US-A- 6 046 310
- US-B1- 6 316 256
- BULFONE-PAUS S ET AL: "AN INTERLEUKIN-2-IGG-FAS LIGAND FUSION PROTEIN SUPPRESSES DELAYED-TYPE HYPERSENSITIVITY IN MICE BY TRIGGERING APOPTOSIS IN ACTIVATED T CELLS AS A NOVEL STRATEGY FOR IMMUNOSUPPRESSION" TRANSPLANTATION, WILLIAMS AND WILKINS, BALTIMORE, MD, US, Bd. 69, Nr. 7, 15. April 2000 (2000-04-15), Seiten 1386-1391, XP002942713 ISSN: 0041-1337
- FANSLOW W C ET AL: "Structural characteristics of CD40 ligand that determine biological function." SEMINARS IN IMMUNOLOGY. UNITED STATES OCT 1994, Bd. 6, Nr. 5, Oktober 1994 (1994-10), Seiten 267-278, XP002264604 ISSN: 1044-5323
- BAUM PETER R ET AL: "Molecular characterization of murine and human OX40/OX40 ligand systems: identification of a human OX40 ligand as the HTLV-1-regulated protein gp34" EMBO (EUROPEAN MOLECULAR BIOLOGY ORGANIZATION) JOURNAL, Bd. 13, Nr. 17, 1994, Seiten 3992-4001, XP002264605 ISSN: 0261-4189
- HOLLER N ET AL: "Development of improved soluble inhibitors of FasL and CD40L based on oligomerized receptors" JOURNAL OF IMMUNOLOGICAL METHODS, ELSEVIER SCIENCE PUBLISHERS B.V.,AMSTERDAM, NL, Bd. 237, Nr. 1-2, April 2000 (2000-04), Seiten 159-173, XP004192503 ISSN: 0022-1759
- HOLLER NILS ET AL: "Two adjacent trimeric Fas ligands are required for Fas signaling and formation of a death-inducing signaling complex" MOLECULAR AND CELLULAR BIOLOGY, AMERICAN SOCIETY FOR MICROBIOLOGY, WASHINGTON, US, Bd. 23, Nr. 4, Februar 2003 (2003-02), Seiten 1428-1440, XP002258597 ISSN: 0270-7306
- BODMER J-L ET AL: "The molecular architecture of the TNF superfamily" TIBS TRENDS IN BIOCHEMICAL SCIENCES, ELSEVIER PUBLICATION, CAMBRIDGE, EN, Bd. 27, Nr. 1, 1. Januar 2002 (2002-01-01), Seiten 19-26, XP004332356 ISSN: 0968-0004
- SCHNEIDER P ET AL: "APOPTOSIS INDUCED BY DEATH RECEPTORS" PHARMACEUTICA ACTA HELVETIAE, XX, XX, Bd. 74, Nr. 2/3, März 2000 (2000-03), Seiten 281-286, XP001121938 ISSN: 0031-6865

## Beschreibung

Aus dem Stand der Technik ist eine Vielzahl von Rezeptoren bekannt, die der Klasse der TNF-Rezeptoren angehören und jeweils mit mindestens einem TNF-Liganden als physiologischem Liganden interagieren. Bei den Rezeptoren der TNF-Rezeptorfamilie handelt es sich um Typ I Membranproteine (Nagata et al, Science, 267: 1449, 1995). Ein Beispiel eines gut untersuchten TNF-Rezeptor-TNF-Ligandensystems ist der Fas-Rezeptor (Fas, FasR, CD95), der mit dem natürlichen Liganden FasL in Interaktion tritt und auf diesem Weg ein intrazelluläres Signal auslöst. Insbesondere die Bedeutung des FasL/FasR-Systems für den zellspezifischen Zelltod ist eingehend untersucht worden. Der Fas-Ligand der Ratte (Suda et al, Cell 75: 1169, 1993; Lynch et al, Immunity 1: 131, 1994) und die humane Form (Takahashi et al, International Immunology 6: 1567, 1994) sind auf der cDNA-Ebene kloniert worden. FasL gehört als Mitglied der Familie der TNF-Liganden zur Kategorie der Typ II Membranproteine, d.h. FasL weist eine extrazelluläre carboxyterminale Domäne und eine intrazelluläre aminoterminale Domäne auf. In die Familie der TNF-Liganden gehören bspw. weiterhin die Proteine TNFa (Tumomekrosefaktor a) oder TNFβ (Tumornekrosefaktor β, Eck et al, Journal Biological Chemistry 264: 17595, 1989). Die TNF-Liganden binden jeweils an ihren physiologischen TNF-Rezeptor. Weitere Beispiele von derartigen Liganden aus dem Stand der Technik sind OX40L (bindet an OX40R), CD27L (bindet CD27R), CD30L (bindet an CD30R), RANKL (bindet an RANK-R), CD40L (bindet CD40R), TRAIL (bindet an TRAIL-R1, R2, R3 oder R4) oder TWEAK (bindet an Fn14).

Die nativen Formen der Liganden aus der Typ II Membranpmteinfamilie sind jedoch als solche für den medizinischen Einsatz nicht geeignet. Als Membranproteine können sie als solche nicht verabreicht werden, insbesondere auch auf Grund der hydrophoben Transmembran-Domäne. Es wurde daher im Stand der Technik versucht, solche Fragmente von TNF-Liganden zur Verfügung zu stellen, die die physiologische Wirkung noch aufweisen könnten, ohne jedoch die intrazellulären Abschnitte oder die Transmembran-Domäne aufzuweisen. Bspw. wurden in vitro und in vivo Experimente mit FasL-Fragmenten durchgerührt, die ausschließlich Bereiche der nativ extrazellulär angeordneten FasL-Domänen aufwiesen (sFasL, "soluble FasL", lösliches FasL). Derartige Proteinfragmente konnten jedoch die physiologische Funktion der Liganden, insbesondere im Falle von FasL, nur unzureichend erfüllen, wobei tw. sogar unerwünschte inverse Effekte der löslichen extrazellulären FasL-Domäne gegenüber dem unter physiologischen Bedingungen in aktiver Form offenbar als Trimer vorliegenden FasL-Transmembranprotein beobachtet werden mußten.

US 6 316 256 beschreibt Fusionsproteine die einen Teil eines Fas-Liganden sowie eine Fc Region enthalten. US 6,046,310 beschreibt ebenfalls Fc:Fas-Ligand Fusionsproteine. Bulfone-Paus et al. (Transplantation, 2000, 7 :1386-1391) beschreiben ein IL-2-IgG-Fas-Ligand Fusionsprotein. Fanslow et al. (Seminars in Immunology, 1994, 5:267-278) beschreiben ein CD40L-Fc Fusionsprotein. Baum et al. (EMBO J., 1994, 13:3992-4001) beschreibt ein lösliches murines OX40L-Fc Fusionsprotein. WO 01/83525 beschreibt Fusionsproteine die einen Fc Abschnitt und einen Abschnitt eines Liganden der TNF Familie enthalten. WO01/49866 beschreibt Fusionsproteine die einen Abschnitt eines TNF-cytokins und einen multimerisierenden Abschnitt eines Proteins ausgewählt aus der Gruppe bestehend aus der Familie des C1q-Proteine und der Collectine enthält. Holler et al. describes Fas :COMP and CD40 :COMP Fusionsproteine.

Die obengenannten Dokumente beschreiben kein Fusionsprotein gemäss Anspruch 1 der vorliegenden Anmeldung.

Aufgabe der vorliegenden Erfindung ist es daher solche Sequenzen zur Verfügung zu stellen, die nicht nur die physiologischen Effekte von TNF-Liganden, imitieren und abbilden, sondern auch löslich sind und sich daher für den Einsatz als Pharmazeutikon, insbesondere auch zur Herstellung eines Arzneimittels, eignen.

Erfindungsgemäß werden Fusionskonstrukte, d.h. sowohl Nukleotid-Sequenzen als auch die aus den Nukleotid-Sequenzen abgeleiteten Proteinsequenzen zur Verfügung gestellt, die es insbesondere auch erlauben, den Phänotyp genetisch bedingter Erkrankungen aufzuheben. Fusionskonstrukte erfindungsgemäßer Art weisen eine Struktur, wie gemäß Anspruch 1 auf. Der im Fusionskonstrukt N-terminal gelegene Abschnitt des Immunglobulins (Ig), also die Komponente (A), weist erfindungsgemäß nicht die für Immunglobuline charakteristische variable Region, die für die Antigen-Erkennung verantwortlich ist, auf, sondern ausschließlich Domänen oder Abschnitte von Domänen der konstanten Region von Immunglobulinen, beispielsweise die Domäne(n) CH₁,CH₂ und/oder CH₃. Es können also erfindungsgemäß Abschnitte der vorgenannten CH-Domänen erfindungsgemäß als Komponente (A) miteinander verbunden werden, also bspw. die Domäne CH₁ und die Domäne CH₃, wobei die Komponente (A) ihre Fähigkeit, an physiologische Fc-Rezeptoren zu binden, erfindungsgemäß bei einer Verwendung zur Herstellung eines Arzneimittels zur Behandlung in utero nicht verlieren sollte. Im Falle einer Verwendung zur Herstellung eines Arzneimittels zur postnatalen Behandlung stellt sich die Situation ggf. so dar, dass es ein Vorteil wäre, wenn die erfindungsgemäßen Konstrukte nicht an Fc-Rezeptoren binden würden. Bei einer Verwendung zur Herstellung eines Arzneimittels zur postnataler .Therapie kann es also bevorzugt sein, wenn die Komponente (A) gerade nicht mehr die Eigenschaft aufweist, funktional an Fc-Rezeptoren zu binden. Ein derartiger Funktionsverlust kann bspw. durch Insertion, Deletion oder Substitution funktionaler Fc-Sequenzen erreicht werden.

Die als Komponente (A) im erfindungsgemäßen Fusionskonstrukt vorliegende Immunglobulin-Sequenz aus der Fc-Region ist zu einer Dimerisierung mit einem anderen Fusionskonstrukt in der Lage. Vorzugsweise ist die Komponente (A) zur Dimerisierung mit einem vorzugsweise identischen erfindungsgemäßen Fusionskonstrukt, alternativ aber auch mit einem anderen erfindungsgemäßen Fusionskonstrukt, bspw. einem Fusionskonstrukt mit einer anderen Komponente (B), befähigt. Die Dimerisierung kann durch die nativ zwischen der Domäne CH₁ und CH₂ gelegene "Hinge"-Region über eine Disulfidbrücke erfolgen oder aber auch über eine artifiziell eingeführt Sequenz (oder bspw. auch Substitution/Insertion eines Cysteins), die kovalent (Disulfidbrücke) oder non-kovalent (bspw. Leucin-Zipper oder andere zur Dimerisierung geeignete Sequenzabschnitte) dimerisieren kann.

In einer bevorzugten Ausführungsform wird die konstante Region des Antikörpers im Fusionsprotein humanen Ursprungs sein, beispielsweise vom Antikörper GI2765420 stammen, und der Familie Immunglobuline aus der Immunglobulinklasse IgG angehören, insbesondere aus den Klassen IgG1, IgG2, IgG3 oder IgG4, bevorzugt aus den Klassen IgG2 oder IgG4. Alternativ können auch konstante Regionen von Immunglobulinen der IgG-Klasse anderer Säugetiere zum Einsatz kommen, insbesondere von Nagetieren oder Primaten, aber auch konstante Regionen der Immunglobulinklassen IgD, IgM, IgA oder IgE können erfindungsgemäß eingesetzt werden. Typischerweise werden die im erfindungsgemäßen Konstrukt enthaltenen Antikötperfragmente die Fc-Domäne CH₃ oder Teile hiervon und zumindest einen Teilabschnitte der Fc-Domaine CH₂ umfassen. Wahlweise sind auch erfindungsgemäße Fusionskonstrukte denkbar, die die CH₃-Domäne und die "Hinge"-Region zur Dimerisierung als Komponente (A) aufweisen.

Es können allerdings auch Derivate der nativ auftretenden Immunglobulin-Sequenzen eingesetzt werden, insbesondere solche Varianten, die mindestens eine Substitution, Deletion und/oder Insertionen aufweisen (hier unter dem Begriff "Variante" zusammengefaßt). Typischerweise haben derartige Varianten mindestens 90%, bevorzugt mindestens 95% und ganz besonders bevorzugt mindestens 98% Sequenzidentität mit der nativen Sequenz. Besonders bevorzugte Varianten sind dabei Substitutionsvarianten mit typischerweise weniger als 10, bevorzugt weniger als 5 und ganz besonders bevorzugt weniger als drei Substitutionen gegenüber der jeweiligen nativen Sequenz. Als Substitutionsmöglichkeiten werden als bevorzugt hervorgehoben: Trp durch Met, Val, Leu, Ile, Phe, His, Tyr oder vice versa; Ala durch Ser, Thr, Gly, Val, Ile, Leu oder vice versa; Glu durch Gln, Asp, Asn oder vice versa; Asp durch Glu, Gln, Asn oder vice versa; Arg durch Lys oder vice versa; Ser durch Thr, Ala, Val, Cys oder vice versa; Tyr durch His, Phe, Trp oder vice versa; Gly oder Pro durch eine der anderen 19 nativen Aminosäuren oder vice versa.

Ganz besonders bevorzugt sind solche Modifikationen vorgenannter Art für die Verwendung zur Herstellung eines Arzneimittels zur Behandlung in utero, wenn die Bindung an membranständige Fc-Rezeptoren zumindest nicht beeinträchtigt ist, ggf. aber sogar optimieren. Bei Verwendung erfindungsgemäßer Konstrukte für die Herstellung eines Arzneimittels für die postnatale Therapie ist es hingegen bevorzugt, die Bindungsfähigkeit an Fc-Rezeptoren zu minimieren oder sogar auszuschalten. Da erfindungsgemäße Fusionskonstrukte als Komponente (A) Sequenzen enthalten müssen, die die Fähigkeit zur Bindung an die jeweiligen physiologischen Rezeptoren für den konstanten Abschnitt von Immunglobulinen beibehalten müssen, werden also in dem als Komponente (A) eingesetzten Antikörperfragment eines erfindungsgemäßen Fusionskonstrukts die Aminosäuren insbesondere an den Positionen 230 bis 240, stärker bevorzugt an den Positionen 234 bis 237 der Domäne CH₂ nicht verändert sein, oder nur mit solchen Sequenz-Variationen versehen sein, die das Bindungsverhalten an bspw. Fc-Rezeptoren unbeeinträchtigt lassen. Substitutionen oder Deletionen sind hingegen gegenüber der nativen Sequenz von konstanten Regionen von Immunglobulinen insbesondere an jenen Positionen bevorzugt, die zu Folge haben, daß Glykosilierungsstellen eliminiert oder eingeführt werden, Disulfidbrücken eingeführt oder eliminiert werden, die Stabilität oder Löslichkeit beeinträchtigen oder die Zellmembran-Passage nach Bindung nach Fc-Rezeptoren verbessern. Insbesondere bevorzugt sind solche Varianten, die gegenüber der dreidimensionalen Auffaltung der nativen Sequenz keine oder nur vernachlässigbare Strukturveränderungen aufweisen. Derartige Strukturidentität (und damit Funktionshomologie) kann mit Hilfe der Aufzeichnung entsprechender Spektren, bspw. eines Zirkulardichroismus-Spektrums der nativen Sequenz bzw. der jeweiligen Variante, ermittelt werden. Durch den Spektrenverlauf, insbesondere in einem Messbereich zwischen 190 und 240 nm Wellenlänge, kann die Strukturidentität jederzeit festgestellt werden. Insbesondere wird in diesem Zusammenhang auf die einschlägige Literatur der CD-Meßgeräthersteller (bspw. Jasco, Japan) verwiesen.

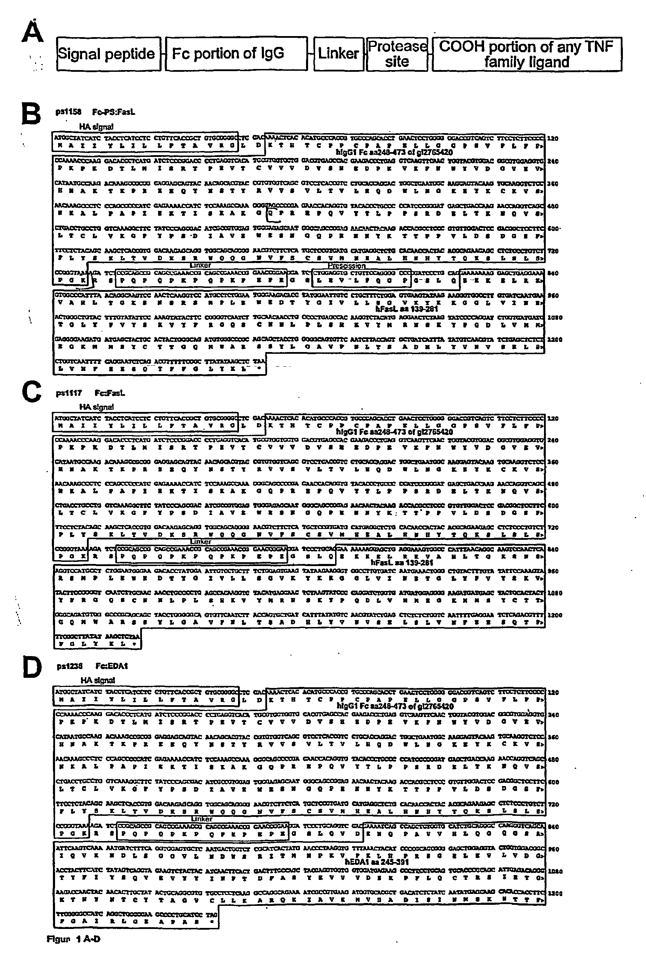

C-terminal vom Immunglobulinfragment enthält ein erfindungsgemäße Fusionskonstrukt typischerweise, aber nicht notwendigerweise, einen Übergangsbereich zwischen den Komponenten (A) und (B), der wiederum eine Linkersequenz enthalten kann, wobei diese Linkersequenz, vorzugsweise eine Peptidsequenz ist. Diese Peptidsequenz kann eine Länge von zwischen 1 und bis zu 70 Aminosäuren, ggf. auch mehr Aminosäure, aufweisen, vorzugsweise 10 bis 50 Aminosäuren und besonders bevorzugt zwischen 12 und 30 Aminosäuren. Beispiele für besonders stark bevorzugte Übergangssequenzen sind in den Figuren 1b bis 1j dargestellt (dort entsprechend markiert). Der Linkerbereich der Übergangssequenz kann von weiteren kurzen Peptidsequenzen, die bspw. DNA-Restriktionsschnittstellen entsprechen können, eingerahmt sein. Alle dem Fachmann aus der Molekularbiologie geläufigen Restriktionsschnittstellen können hierbei zum Einsatz kommen. Als Linkersequenzen kommen vorzugsweise artifizielle Sequenzen in Betracht, die eine hohe Anzahl an Prolin-Residuen aufweisen (beispielsweise an jeder zweiten Position im Linkerbereich) und darüber hinaus vorzugsweise insgesamt hydrophilen Charakter haben. Eine Linkersequenz mit mindestens 30% Anteil an Prolinresten ist bevorzugt. Der hydrophile Charakter kann bevorzugt durch mindestens eine Aminosäure mit positiver Ladung, beispielsweise Lysin oder Arginin, oder negativer Ladung, beispielsweise Aspartat oder Glutamat, hervorgerufen werden. Insgesamt weist der Linkerbereich bevorzugt auch deshalb eine hohe Anzahl an Glycin- und/oder Prolinresten auf, um dem Linkerbereich die erforderliche Flexibilität und/oder Steifigkeit zu verleihen.

Als Linker kommen jedoch auch native Sequenzen in Betracht, beispielsweise jene Fragmente von Liganden der TNF-Liganden-Familie die extrazellulär, jedoch unmittelbar an, d.h. vor der Zellmembran angeordnet sind, ggf. auch nach Substitution, Deletion oder Insertion der nativen Abschnitte. Bei diesen Fragmenten handelt es sich vorzugsweise um die - nach der Transmembran-Region extrazellulär folgenden - 50 AS oder Unterfragmente dieser ersten 50 AS. Bevorzugt sind jedoch mindestens 85% Sequenzidentität dieser Abschnitte mit den entsprechenden natürlichen humanen Sequenzen, ganz besonders bevorzugt mindestens 95% und insbesondere bevorzugt mindestens 99% Sequenzidentität, um die Immunogenizität dieser Linkerbereiche im erfindungsgemäßen Fusionsprotein zu begrenzen und keine körpereigene humorale Abwehrreaktion hervorzurufen. Im Rahmen der vorliegenden Erfindung gilt, daß der Linkerbereich vorzugsweise keine Immunogenizität besitzen sollte.

Alternativ zu Peptidsequenzen, die über amidartige Bindungen mit dem Antikörperfragment und dem TNF-Liganden oder ein Fragment eines solchen TNF-Liganden verbunden sind, können jedoch auch Verbindungen, die nicht-peptidischer Art oder pseudopeptidischer Art sind oder auf nicht-kovalenten Bindungen beruhen, zum Einsatz kommen. Hierbei werden als Beispiele insbesondere N-Hydroxysuccinimidester und heterobifunktionale Linker, wie zum Beispiel N-Succinimidyl-3-(2-pyridyldithio)-proprionat (SPDP) oder ähnliche Crosslinker genannt.

Carboxyterminal vom Übergangsbereich folgt in einem erfindungsgemäßen Fusionsprotein Komponente (B). Bevorzugt sind im Rahmen der vorliegenden Erfindung Fragmente, die insbesondere den äußerst carboxyterminal gelegenen Teil des extrazellulären Abschnitts des TNF-Liganden EDA1 aufweisen. In Hinblick auf die native Sequenz des extrazellulären Abschnittes des vorgenannten Proteins wird auf die entsprechenden Einträge in der Datenbank SwissProt (http://www.expasy.ch/sprot/sprot-top.html) verwiesen.

Im Hinblick auf EDA1 sind Fragmente der Aminosäuren 140 bis 391 oder am N-Terminus stärker verkürzte Fragmente (bspw. 157, 160, 181 oder 182 bis 391), insbesondere 200 bis 391, ganz besonders, 245 bis 391 oder am N-Terminus noch stärker verkürzte Fragmente (bspw. 246 bis 391) bevorzugt. Im N-terminalen Bereich der im erfindungsgemäßen Fusionsprotein als Komponente (B) enthaltenen Sequenzen sind allerdings auch alle zwischen den jeweils oben als bevorzugt genannten Bereichsangaben liegenden Sequenzabschnitte einsetzbar, wobei ein erfindungsgemäßes Fusionsprotein typischerweise C-terminal mit dem nativen C-terminalen Ende des TNF-Liganden EDA1 abschließt. Ggf. können allerdings auch im C-terminalen Bereich des als Komponente (B) eingesetzten extrazellulären EDA1 Fragments im erfindungsgemäßen Fusionsprotein mindestens eine Aminosäure deletiert sein, typischerweise 2 bis 10, in seltenen Fällen auch mehr als 10 Aminosäuren.

Der im erfindungsgemäßen Fusionsprotein enthaltene TNF-Ligand EDA1 kann gleichfalls gegenüber der nativen Sequenz mindestens eine Substitution, Deletion und/oder Insertion ausweisen, um erwünschte biologische Wirkungen zu entfalten, wie zum Beispiel Löslichkeit, Stabilität oder veränderte Immunogenizität Alternativ zur nativen TNF-Ligandensequenz kann erfindungsgemäß als Komponente (B) eine gegenüber der nativen Sequenz typischerweise zu mindestens 75%, vorzugsweise 85% und besonders bevorzugt zumindest 95% sequenzidentische Variante im erfindungsgemäßen Fusionsprotein eingesetzt werden, die die gleiche biologische Wirkung entfaltet, insoweit also funktionshomolog ist Derartige Strukturidentität (und damit Funktionshomologie) kann mit Hilfe der Aufzeichnung entsprechender Spektren, bspw. eines Zirkulardichroismus-Spektrums der nativen Sequenz bzw. der jeweiligen Variante, ermittelt werden. Durch den Spekttenverlauf, insbesondere in einem Messbereich zwischen 190 und 240 nm Wellenlänge, kann die Strukturidentität jederzeit festgestellt werden. Insbesondere wird in diesem Zusammenhang auf die einschlägige Literatur der CD-Meßgeräthersteller (bspw. Jasco, Japan) verwiesen.

Besonders bevorzugte Varianten sind dabei Substitutionsvarianten mit typischerweise weniger als 10, bevorzugt weniger als 5 und ganz besonders bevorzugt weniger als drei Substitutionen gegenüber der jeweiligen nativen Sequenz. Als Substitutionsmöglichkeiten werden als bevorzugt hervorgehoben: Trp durch Met, Val, Leu, Ile, Phe, His, Tyr oder vice versa; Ala durch Ser, Thr, Gly, Val, Ile, Leu oder vice versa; Glu durch Gln, Asp, Asn oder vice versa; Asp durch Glu, Gln, Asn oder vice versa; Arg durch Lys oder vice versa; Ser durch Thr, Ala, Val, Cys oder vice versa; Tyr durch His, Phe, Trp oder vice versa; Gly oder Pro durch eine der anderen 19 nativen Aminosäuren oder vice versa.

Ein weiterer Erfindungsgegenstand ist die Verwendung eines erfindungsgemäßen Fusionsproteins zur Herstellung und Konfektionierung eines Arzneimittels zur Reversion genetisch bedingten Erkrankungen. Diese erfindungsgemäße Verwendung ist bei allen Plazentaliern, also Wirbeltieren mit Plazenta, insbesondere in der Human- und Veterinärmedizin, anwendbar. Die erfindungsgemäße Verwendung zur Herstellung eines Arzneimittels ist geeignet, nach Diagnose einer genetisch bedingten Erkrankung im Embryo, beispielsweise durch Chorionbiopsie oder Amnionzentese, oder bei Verdacht auf eine genetisch bedingte Erkrankung beim Embryo aufgrund der genetischen Disposition von Verwandten, insbesondere Vater und/oder Mutter, bereits prophylaktisch, den Embryo zu behandeln und dessen erbgenetischen Phänotyp zu revertieren. Die Verwendung zur Herstellung eines Arzneimittels zur Behandlung erfolgt über ein erfindungsgemäßes Fusionsprotein, so wie oben offenbart, ggf. in entsprechender Konfektionierung, und wird idealer Weise zum frühestmöglichen Zeitpunkt der Schwangerschaft/Trächtigkeit der Mutter bzw. dem Muttertier verabreicht. Vorteilhafterweise erfolgt die Verabreichung eines solchen erfindungsgemäßen Fusionsproteins parenteral, vorzugsweise intravenös oder intraarteriell.

Erfindungsgemäß bindet ein erfindungsgemäßes Fusionsprotein der zuvor beschriebenen Art über seinen Fc-Anteil als Komponente (A) an die Fc-Rezeptoren der Plazenta und erreicht auf diese Weise nach Internalisierung den Embryo, typischerweise über die Plazentagefäße, die den Embryo an den mütterlichen Blutkreislauf anschließen.

Die Dosierung ist abhängig von der genetischen Erkrankung selbst und vom Zeitpunkt der Verabreichung (also vom Entwicklungsstadium des Embryos), wobei das hergestellte Arzneimittel geeignet ist für die Behandlung zum frühestmöglichen Zeitpunkt der Embryonalentwicklung. Das hergestellte Arzneimittel ermöglicht die Verabreichung derartiger erfindungsgemäßer Fc:Ligandenkonstrukte, bspw. Fc:EDA1 oder Fc:EDA2, mindestens einmal, stärker bevorzugt regelmäßig während des ersten, zweiten und/oder dritten Monats der Schwangerschaft/Trächtigkeit, ganz besonders bevorzugt bspw. an jedem zweiten Tag für die Dauer von mindestens 14 Tagen beim Humanembryo, ggf. aber auch in größeren Abständen abhängig von der gewählten Dosierung.

Grundsätzlich ist eine Dosierung eines erfindungsgemäßen Fusionskonstrukts jedoch abhängig von der Behandlungsmethode. Im Falle einer Verwendung zur Herstellung eines Arzneimittels zur der Behandlung der Embryonalentwicklung, sind typischerweise Dosierungen des verabreichten Konstrukts von weniger als einem Zehntel, vorzugsweise weniger als einem Hundertstel und noch stärker bevorzugt weniger als einem Tausendstel der nativen Konzentration des TNF-Liganden im Neugeborenen angeraten. Im Falle einer Verwendung zur Herstellung eines Arzneimittels zur Behandlung des Neugeborenen oder des Kleinkinds mit erfindungsgemäßem Fusionsprotein werden die Dosierungen mindestens 1 /100, stärker bevorzugt mindestens 1/10 (stets bezogen auf die TNF-Ligandenaktivität), der für den gesunden Patienten gleichen Alters typischen Serumkonzentration betragen.

Ganz besonders bevorzugt ist die Verwendung eines erfindungsgemäßen Fusionsprotein für die Konfektionierung als Arzneimittel in einem derartigen Verfahren dann, wenn ein erfindungsgemäßes Fusionskonstrukt, das einen Fc-Anteil am N-Terminus des Fusionskonstrukts und am C-Terminus einen Abschnitt des TNF-Liganden EDA1 als Komponenten (B) trägt, insbesondere von EDA1 humanen Ursprungs, vor allem einem extrazellulären Abschnitt dieser Proteine, der sich von mindestens Aminosäure 200 bis zur Aminosäure 391 (EDA1) erstreckt, ganz besonders bevorzugt von Aminosäure 245 bis 391 (EDA1) verabreicht wird. Noch stärker bevorzugt sind solche erfindungsgemäßen Fusionskonstrukte für eine entsprechende Verwendung, wie sie in den Figuren 1d dargestellt sind.

Diese erfindungsgemäßen Fusionskonstrukte sind dazu geeignet in der Veterinärmedizin den Phänotyp der Nachkommenschaft von Tabby-Mäusen durch Behandlung während der Schwangerschaft/Trächtigkeit zu revertieren Entsprechend ist die Verwendung erfindungsgemäßer Fusionsproteine für die Herstellung von Arzneimittel, die verfahreastauglich sind, auch zur therapeutischen Behandlung der analogen Humanerkrankung, also einer Erberkrankung, die auf dem gleichen genetischen Defekt basiert, nämlich der X-gekoppelten hypohydrotischen ektodermalen Dysplasie (XLHED, Christ-Siemens-Touraine-Syndrom), der häufigsten Form ektodermaler Dysplasien, einsetzbar. XLHED beruht auf einem Defekt des Gens ED1, das in der Humanmedizin mit folgendem klinischen Krankheitsbild korrespondiert Kleinwuchs, Demenz unterschiedlichen Ausmaßes, Sattelnase, Anhydrosis, fehlende Zähne oder abnorme Entwicklung der Zähne, Hypertrichosis oder Alopezie, oder Fehlfunktion bzw. Fehlen ekkriner Schweißdrüsen zu nennen, weswegen die betroffenen Patienten gegenüber Hyperthermie ausgesprochen empfindlich sind. Die erfindungsgemäßen Arzneimittel sind neben XLHED auch für andere ektodermale Dysplasien geeignet, denen andere erbgenetische Ursachen zugrunde liegen.

Ein weiterer Gegenstand der vorliegenden Erfindung ist somit die Verwendung erfindungsgemäßer Fusionskonstrukte zur Herstellung eines Arzneimittels zur Behandlung genetisch bedingter Erkrankungen bei Mensch oder Tier, Insbesondere kommen erfindungsgemäße Fusionskonstrukte, insbesondere Konstrukte, die Abschnitte des TNF-Liganden EDA1 enthalten, beispielsweise die Fusionskonstrukte gemäß Figur 1d, generell zur Herstellung eines Arzneimittels zur Behandlung von genetisch bedingten Hautanomalien, insbesondere Anomalien der Hautstruktur oder auch Anomalien ektodermaler Strukturen, wie beispielsweise Anomalien des Haares, der Zähne oder der Drüsen (An- oder Dyshydrosen verschiedener Genese) in Betracht. Als Beispiel sei die Verwendung erfindungsgemäßer Fusionsproteine zur Herstellung eines Arzneimittels im kosmetischen Bereich, z.B. zur Behandlung von Alopezie (erworben, bspw. in Form der Alopecia areata, Alopezia atrophicans, Alopecia seborrhoica oder Alopecia praematura) oder angeboren (vor allem als Syndrom der Anhidrosis hypotrichotica) oder Hirsutismus genannt. Erfindungsgemäße Fusionskonstrukte können allerdings auch ganz allgemein zur Herstellung eines Arzneimittels zur Behandlung aller Formen ektodermaler Dysplasien eingesetzt werden. Daher sind erfindungsgemäße Fusionskonstrukte, insbesondere jene unter Beteiligung von EDA1 besonders geeignet zur Herstellung eines Arzneimittels zur Behandlung der Stimulation des Haarwachstums, der Inhibition des Haarwachstums, der Behandlung von Wachstumsstörungen der Zähne oder auch zur Behandlung von Störungen der Drüsenfunktion, einschließlich der Schweiß- und/oder Talgdrüsen, oder auch zur Haarfollikelbildung oder zur Wundheilung, insbesondere zur Wundheilung von Patienten zur Sicherstellung entsprechenden Haarwachstums auf der betroffenen Hautpartie. Die Verwendung derartiger Fusionskonstrukte zur Herstellung eines Arzneimittels zur Verabreichung an den Patienten, vorzugsweise postnatal, ganz besonders bevorzugt in der Humantherapie innerhalb des ersten Lebensjahres, noch stärker bevorzugt innerhalb der erstens 6 Lebensmonate, noch stärker bevorzugt innerhalb der erstens zwei Lebensmonate, noch stärker bevorzugt innerhalb der ersten 2 Lebenswochen, noch stärker bevorzugt innerhalb der erstens 3 Tage nach der Geburt, oder zur Verabreichung an die schwangere Mutter (oder das trächtige Muttertier), bspw. Injektion in utero, erfolgen. Die erfindungsgemäßen Fusionskonstrukte können also auch zur Herstellung eines Arzneimittels zur Behandlung der vorgenannten Syndrome, genetischen Erkrankungen oder Störungen während der Embryonalentwicklung durch Verabreichung an die Mutter bzw. das Muttertier eingesetzt werden, ausgenommen Verfahren bei denen die genetische Identität der Keimbahn des menschlichen Lebewesens verändert wird.

Im Falle der Herstellung eines Arzneimittels zur Behandlung von insbesondere Alopezie oder insbesondere zur Wundheilung, bspw. nach Hautverbrennungen oder entzündlichen Hauterkrankungen, bspw. bei der Behandlung neurodermitischer oder atopisch betroffener Hautpartien, insbesondere der Kopfhaut, durch die Verwendung erfindungsgemäßer Konstrukte kommt auch die Verabreichung derselben, ggf. unter Verwendung weiterer Hilfs-, Träger- oder Zusatzstoffe, ggf. auch weiterer Wirkstoffe(z.B. bei der Bwehandlung von Alopezie: Relaxin, Anti-androgen wirkende Substanzen, bspw. Finasterid, SKL-105657, Östrogen, Cyproteronacetat, Spironolactor, Flutamid, Minoxidil und/oder RU58841) beim Erwachsenen in Betracht, bspw. durch intravenöse oder intraarterielle oder subkutane Verabreichung, nach entsprechender Aufbereitung (Konfektionierung) jedoch auch oral oder als Creme, Lotion, Salbe zur topischen Anwendung.

Als weiteres Einsatzgebiet erfindungsgemäßer Konstrukte kommt deren Verwendung bei der Herstellung von Hautgewebe ex vivo in Betracht Bspw. können erfindungsgemäße Gegenstände, bspw. in Form der Proteine, der Nukleinsäuren oder der Expressionsvektoren, eingesetzt werden, um die natürliche Funktionalität der Haut von Patienten mit Hauterkrankungen (bspw. Diabetes-Geschwüren) oder Verbrennungen, unter Bildung von ekkrinen Drüsen, Haarfollikeln und Haaren, zu (re)generieren. Hierbei werden erfindungsgemäße Gegenstände Zellen oder Geweben in vitro zugesetzt Bspw. können erfindungsgemäße Gegenstände im Rahmen des traditionellen Verfahrens des "skin grafting" zum Einsatz kommen, also bspw. kann autologes, nicht erkranktes Hautgewebe des Patienten, allogenes Hautgewebe Verstorbener oder von Fremdspendem oder Tiergewebe vor dessen Transplantation durch Behandlung mit erfindungsgemäßen Fusionskonstrukten zur Bildung von Haarfollikeln, ekkrinen Drüsen angeregt werden, bspw. im Rahmen einer Vorbehandlung oder im Rahmen extrakorporaler Kultivierung. Allerdings können auch multipotente Vorläuferzellen aus nicht erkranktem Hautgewebe (oder anderem Gewebe) des Patienten selbst isoliert werden Diese Zellen werden durch den Zusatz entsprechender Differenzierungsfaktoren zu Hautgewebe ausgebildet. Diese multipotenten Vorläuferzellen können durch entsprechende Transfektionen mit erfindungsgemäßen Sequenzen oder Expressionsvektoren transfiziert werden oder es können ihnen bei der Bildung von Hautgewebe Fusionskonstrukte zugesetzt werden. Damit können erfindungsgemäße Gegenstände beim sog. "tissue engineering" (Gewebekultivierung, Gewebezüchtung) zum Einsatz kommen, bevor das kultivierte Gewebe dem Patienten retransplantiert wird. Grundsätzlich wird hierzu wird das zu transplantierende Gewebe (Vorläuferzelle, Stammzelle, Gewebezelle (insbesondere Kerationocyten der Epidermis) oder nicht menschliche embryonale Stammzelle (autolog oder allogen) zunächst isoliert, dann in einem offenen System auf einer Matrix (natürlich, synthetisch oder xenogenisch) oder einem bio-degradablen Gerüst unter Zusatz ausreichender Versorgung der Zellen mit Nährstoffen, Sauerstoff, Wachstumsfaktoren und erfindungsgemäßen Konstrukten und unter Eliminierung von schädlichen Metaboliten gezüchtet. Damit ist ein weiterer Gegenstand der vorliegenden Erfindung ein in vitro Verfahren zur Züchtung von Hautgewebe, das ekkrine Drüsen und natürlichen Haarwuchs aufweist.

Ein weiterer Gegenstand der vorliegenden Erfindung ist auf die den erfindungsgemäßen Fusionsproteinen zugrundeliegenden rekombinanten DNA-Konstrukte gerichtet. DNA-und Proteinkonstrukt werden im Rahmen der vorliegenden Erfindung gemeinsam unter dem Begriff "Fusionskonstrukt" behandelt So kann beispielsweise ein erfindungsgemäßes Nukleotidkonstrukt für die Domänen CH2 und CH3 von IgG als Komponente (A) und EDA1 kodieren, ggf. verbunden durch einen Übergangsbereich, enthaltend einen Linker. Die zugrundeliegenden erfindungsgemäßen rekombinanten Nukleotid-Sequenzen codieren demnach für all jene erfindungsgemäßen Proteinfusionskonstrukte, die zuvor offenbart worden sind. Insbesondere bevorzugt sind die Nukleotidsequenzen der in Figur 1d offenbarten Fusionskonstrukte. Die erfindungsgemäßen Nukleotidkonstrukte sind mit Hilfe von Klonierungsverfahren oder über chemische Synthese erhältlich, und zwar als cDNA, genomische DNA oder in synthetischer Form, wobei eine Vielzahl von Methoden aus dem Stand der Technik in Betracht kommt Im Hinblick auf die Sequenzen der Domäne des Fc-Anteils von Immunglobulinen, die in einem erfindungsgemäßen Nukleotidkonstrukt auftreten können, wird beispielsweise auf die Veröffentlichung "Sequences of Proteins of Immunologic Interest", 1st Edition, Kabat et al., US Department of Health and Human Services, 1991" verwiesen, wobei diese Veröffentlichung vollinhaltlich der vorliegenden Offenbarung zugerechnet wird.

Die Sequenzen, die in einem erfindungsgemäßen Nukleotidkonstrukte enthalten sind, werden typischerweise in Segmenten vorliegen, die durch Restriktion und Ligation zusammengefügt werden. Typischerweise wird eine erfindungsgemäße Nukleotidsequenz von Expressionkontrollsequenzen, die operabel mit der erfindungsgemäßen NukleotidSequenz für ein erfindungsgemäßes Fusionsprotein verbunden sind, enthalten, einschließlich Promotoren, Ribosomen-Bindungsstellen, Polyadylierungs- und/oder Transkriptionsterminationsstellen, und ggf. Enhancer. Ein erfindungsgemäßes Fusionsprotein kann durch Transfektion derartiger regulatorische Elemente enthaltender DNA-Segmente exprimiert werden, z.B. auf Plasmidvektoren, in bakterielle Zellen, Hefezellen, Pflanzenzellen, Insektenzellen und vorzugsweise Säugerzellen, und zwar bspw. unter Verwendung der Calciumphosphat-Methode, Elektroporation oder ähnlicher Techniken. Zur Expression in eukaryotischen Zellen wird der Promotor und ggf. die Enhancer-Sequenz von beispielsweise Immunglobulin-Genen, SV40, Retroviren, Cytomegalovirus, dem Elongationsfaktor 1α etc. eingesetzt werden. Bevorzugte Wirtszellinien schließen CHO-Zellen, COS-Zellen, Hela-Zellen, NIH3T3-Zellen und verschiedene Myeloma- oder Hybridoma-Zellinien, einschließlich des P2/0 und NS/0, ein. Sowohl derartige erfindungsgemäße DNA-Sequenz, die für ein erfindungsgemäßes rekombinantes Fusionsprotein codieren als auch Expressionsvektoren, die eine derartige erfindungsgemäße DNA-Sequenz enthalten, als auch Wirtszellen, die mit einem derartigen erfindungsgemäßen Expressionsvektor transfiziert sind, sind Gegenstand der vorliegenden Erfindung.

Der Plasmidvektor mit erfindungsgemäßem DNA-Konstrukt wird im allgemeinen einen selektierbaren Marker enthalten, wie z.B. gpt, neo, hyg oder DHFR und ein amp, tet, kan etc. Gen zur Expression in E.coli. Eine Vielzahl von Plasmidvektoren, die geeignet sind zur Expression von heterologen Proteinen, ist im Stand der Technik verfügbar. Ein erfindungsgemäßes Fusionsprotein enthält vorteilhafter Weise eine N-terminale Sequenz, beispielsweise eine Sequenz aus Hämagglutinin und/oder Tag Sequenzen und/oder oder mindestens eine weitere "LEADER"-Sequenz am Aminoterminus des Fusionsproteins, um die Sekretion der Fusionsproteine aus der Zelle zu erleichtern, insbesondere die Passage über das ER in den extrazellulären Raum bzw. in das Medium.

Verfahren zur Konstruktion von erfindungsgemäßen Nukleotidkonsumhen, die für erfindungsgemäße Proteinkonstrukte codieren, deren Verbindung mit Expressionskontrollsequenzen und/oder die Insertion im Plasmide, die Transfektion in Zellen und die Selektion und wahlweise Genamplifikation der Fusionsprotein exprimierenden Zellinie kann durch Methoden durchgeführt werden, die aus der Gentechnologie bekannt sind, einschließlich Restriktionsenzymverdauung, Ligation Oligonukleotidsynthese und PCR-Reaktion (Sambrook et al, Molekular Cloning: Laboratory Manual, 3rd Edition, Cold Spring Harbor Laboratory Press, 2001), wobei auf diese Veröffentlichung diesbezüglich vollinhaltlich Bezug genommen wird.

Eine transfizierte Zellinie, die ein erfindungsgemäßes Fusionsprotein exprimiert und sekretiert, wird selektiert und kann ggf. in serumfreien Medium (Hybridoma SFM) aufgezogen werden, indem sie durch abnehmende Konzentration von Serum gerührt wird und kann schließlich subkloniert werden. Erfindungsgemäßes Fusionsprotein kann nach der Sekretion gereinigt werden (aus vorzugsweise serumfreiem Medium), indem die exprimierende Zellinie aufgezogen worden ist Standardverfahren zur Proteinreinigung schließen die Filtration, Präzipitation, Protein A-Affinitätschromatographie, Gelfiltration, Ionenaustauschchromatographie, elektrophoretische Methoden oder ähnliches ein. Im wesentlichen reine Fraktionen des Fusionsproteins von mindestens 90 bis 95 % Homogenität und vorzugsweise von mindestens 98 bis 99 % oder höherer Homogenität werden dann bevorzugt zum pharmazeutischen Gebrauch eingesetzt.

Die Herstellung eines Arzneimittels, das das erfindungsgemäße Fusionsprotein enthält, wird typischerweise die Formulierung in einem pharmazeutisch akzeptablen Trägermaterial einschließen. Bei diesem Trägermaterial wird es sich typischerweise um wässrige Trägermaterialien handeln, wobei Wasser zur Injektion (WFI) oder Wasser gepuffert mit Phosphat, Zitrat oder Acetat etc. verwendet wird, und ein pH von typischerweise 5,0 bis 8,0, vorzugsweise 6,0 bis 7,0 eingestellt wird. Der pharmazeutisch akzeptable Träger wird zusätzlich vorzugsweise Salzbestandteile enthalten, z.B. Natriumchlorid, Kaliumchlorid oder andere Komponenten, die die Lösung isotonisch machen. Weiterhin kann der Träger zusätzliche Komponenten, wie z.B. humanes Serum Albumin, Polysorbat 80, Zucker oder Aminosäuren, enthalten. Eine derartige erfindungsgemäße Zusammensetzung kann in Form einer Injektion oder auch als Kit in einer entsprechend geeigneten Kombination mit einem pharmazeutisch akzeptablen Trägermaterial oder einem Mediator, wie z.B. sterilisierte Wasser oder physiologischer Kochsalzlösung, pflanzlicher Öle, mineralischer Öle, höherer Alkohole, höherer Fettsäure, nicht toxischer organischer Lösungsmittel kombiniert werden und ggf. mit weiteren Additiven versehen sein. Als weitere Additive kommen bspw. Füllstoffe (bspw. Saccharide (Laktose, Sucrose, Mannitol oder Sorbitol), Calciumphosphat, Cellulose oder deren Derivate), Bindemittel (bspw. Stärke, Gelatine, Polyvinylpyrtolidon), Stabilisatoren, Präservative, Antiadsorbanzien, oberflächenaktive Substanzen, Farb- oder Geschmacksmittel, Emulgatoren, Pufferagenzien, isotonische Agenzien, Antioxidanzien, Schmerzmittel oder ähnliches in Betracht. Vorzugsweise werden bspw. Natriumalginat, Polyethylenglykol und/oder Titandioxid in die Formulierungen eingearbeitet. Entsprechende Formulierungen eines Arzneimittels sind bei Remington (1980) offenbart, worauf diesbezüglich verwiesen wird.

Die Konzentration des erfindungsgemäßen Fusionsproteins in derartigen Formulierungen kann innerhalb eines weiten Bereichs von 0,001 bis 10 mg pro ml, vorzugsweise in einem Bereich von 0,5 bis 5 mg/ml, variieren. Die Formulierung wird vorzugsweise parenteral, also beispielsweise intravenös, intraarteriell subkutan, intramuskulär dem Patienten oder der schwangeren Mutter injiziert. Alternativ kann bei Behandlung durch ein erfindungsgemäßes Verfahren auch direkt die Formulierung in das Plazentagefäßsystem des Embryos eingeführt werden oder in die Fruchtblase injiziert werden.

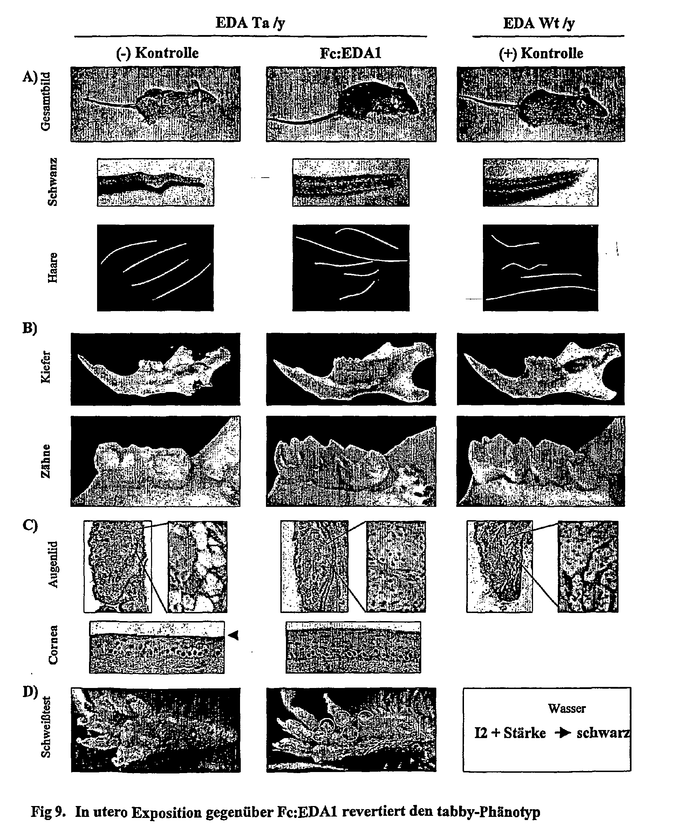

Die vorliegende Erfindung wird durch die nachfolgenden Figuren näher erläutert:

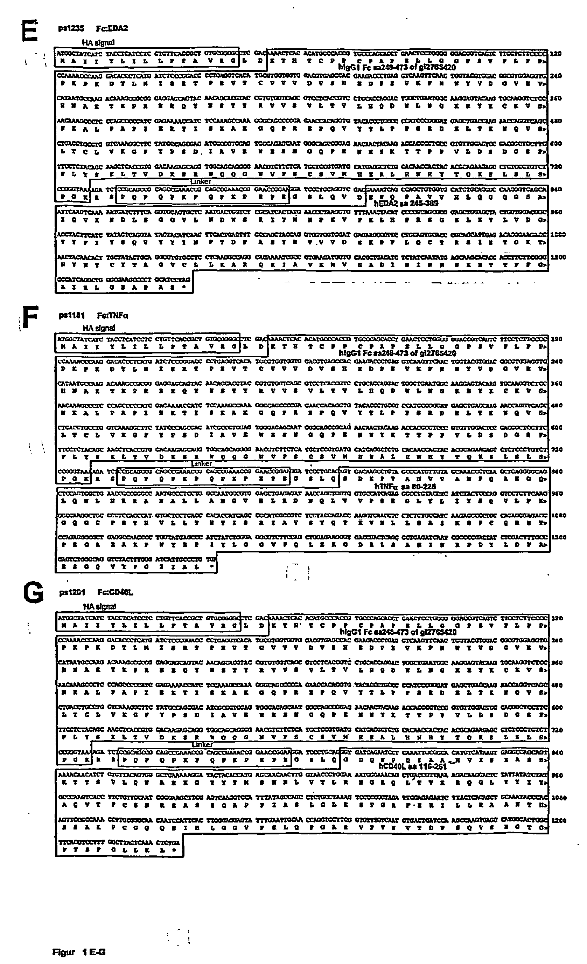

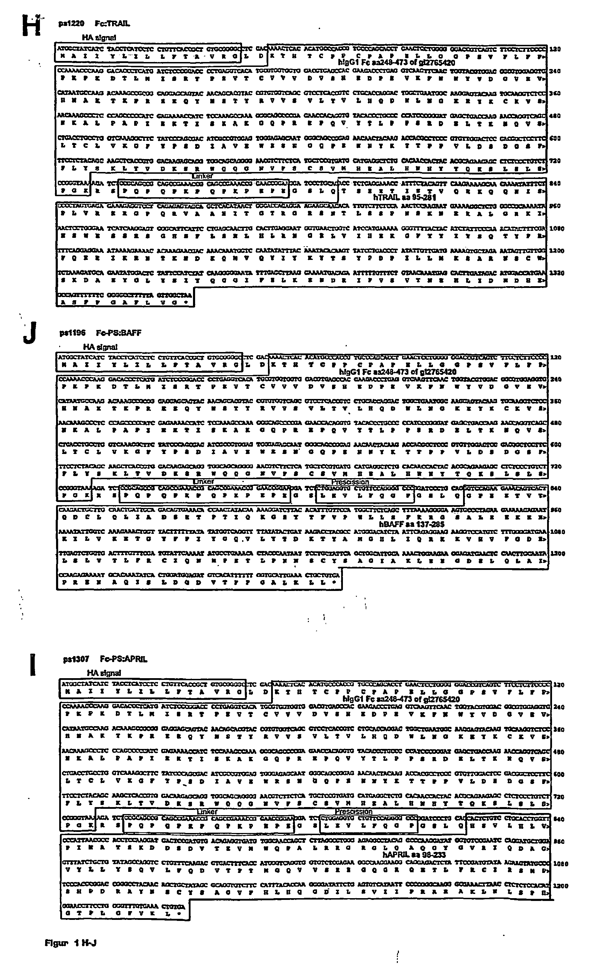

Figur 1 zeigt die Nukleotid- und Aminosäuresequenzen von Fc:Ligand-Konstrukten.
Figur 1a stellt dabei beispielhaft schematisch die Sequenzabschnitte von Fc:Ligand-Konstrukten dar. Im N-terminalen Bereich eines von einer zugrundeliegenden Nukleotidsequenz kodierten Fusionsproteins befindet sich typischerweise ein Signalpeptid, gefolgt von einem Fc-Abschnitt von IgG (Komponente (A)), gefolgt von einem (optionalen) Linkerbereich, weiter C-terminal von einer (optionalen) Protease-Schnittstellensequenz und weiter C-terminal (und am C-Terminus eines erfindungsgemäßen Fusionskonstrukts) von einer Sequenz, die typischerweise eine Teilsequenz eines Mitglieds der TNF-Ligandenfamilie darstellt (Komponente (B)). Diese als Komponente (B) dienende Teilsequenz schließt wiederum typischerweise den C-terminalen Teil des extrazellulären Abschnitts eines Liganden aus der TNF-Familie ein.
   Die Teilfiguren 1b bis 1j zeigen spezifische Sequenzen von Konstrukten unter Verwendung verschiedener Mitglieder der Familie der TNF-Liganden, nämlich FasL (Figur 1b und 1c, mit und ohne Proteaseschnittstelle), EDA1 und EDA2 (Figur 1d und 1e), TNFa (Figur 1f), CD40L (Figur 1g), TRAIL (Figur 1h), BAFF (Figur 1j) und APRIL (Figur 1i). Die Teilsequenzen der vorgenannten Mitglieder der TNF-Ligandenfamilie sind sämtlich humanen Ursprungs und weisen jeweils die C-terminal gelegenen extrazellulären Sequenzabschnitte der nativen Liganden bis zum nativen C-Terminus auf. Die N-terminal gelegene Fc-Domine (Komponente (A)) ist mit dem im Fusionsprotein C-terminal gelegenen Abschnitt des TNF-Liganden (Komponente (B)) über eine Linkerregion mit den Aminosäuren PQPQPKPQPKPEPE verbunden. Darüber hinaus enthalten die in Figur 1b, 1i und 1j dargestellten Sequenzen von erfindungsgemäßen Fusionskonstrukten eine Protease-Schnittstelle, jeweils mit der Sequenz LEVLFQGP, so daß die Protease PreSCISSION (Amersham-Pharmacia) die mit einer derartigen Protease-Schnittstelle ausgestalteten Fusionskonstrukte schneiden kann und damit die Immunglobulin-Domäne des Fusionskonstrukts von dem TNF-Ligandenabschnitt abgetrennt werden kann. Als Sequenz einer Protease-Schnittstelle kommen jedoch alle mindestens 4 AS langen Sequenzen in Betracht, die von einer beliebigen Protease, bspw. einer Cystein- oder einer Aspartatprotease, erkannt werden können. Zwischen der Fc-Domäne und dem Linker bzw. dem Linker und der TNF-Ligandendomäne können weitere, typischerweise kurze, d.h. zwei bis acht Aminosäuren lange Sequenzen enthalten sein. Die Sequenzen der Teilfiguren 1b bis 1j enthalten zwischen Fc-Abschnitt und Linker die Aminosäuren ARG-SER und in den Teilfiguren 1c, 1d, 1e, 1f, 1g und 1h befindet sich N-Terminal von der Komponente (B) zumindest das Tetrapeptid GSLQ, ggf. C-terminal von Q verlängert um weitere Aminosäuren. Jene Fusionskonstrukte, die auch eine Protease-Schnittstelle aufweisen, also die Fusionskonstrukte der Teilfiguren 1b, 1i und 1j enthalten das vorgenannte Tetrapeptid C-terminal von der Protease-Schnittstelle. Linker und Protease-Schnittstelle werden typischerweise auch durch mindestens zwei Aminosäuren voneinander getrennt, figurengemäß durch das Dipeptid GS. Der gesamte Bereich zwischen der Komponete (A) und der Komponente (B) wird als Übergangsbereich bezeichnet
   Alle Fusionskonstrukte der Figuren 1b bis 1j weisen am N-Terminus, also N-terminal von der Fc-Domäne ein Pentadekapeptid auf (MAIIYLILLFTAVRG), das in den dargestellten Fällen einer Hämagglutinin-Sequenz entspricht Verbunden ist diese Signalsequenz in allen dargestellten Fällen über das Dipeptid LD mit dem Fc-Abschnitt des Fusionskonstrukts (Komponente (A)).
   Die nicht eingerahmten Verbindungssequenzen der Figuren 1b bis 1j entsprechend typischerweise Restriktionsenzymschnittstellen, mit deren Hilfe die einzelnen Sequenzkomponenten zusammengefügt werden.
   Alle in den Teilfiguren dargestellten Fusionskonstrukte wurden in einem PCR-3-Säugetierexpressionsvektor (Invitrogen) kloniert.

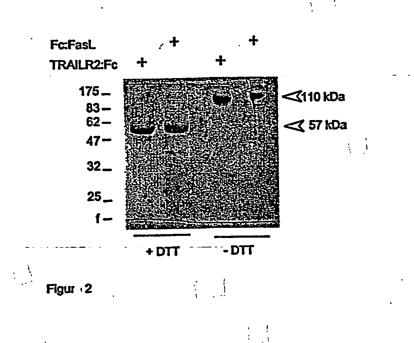

Figur 2 zeigt eine Westesn-Blot-Darstellung, in der die Aufreinigung von Fusionskonstrukt Fc:FasL dargestellt ist. Das aufgereinigte Protein (5 , µg/Spur) wurde auf 12%-SDS-Page unter reduzierenden (+DTT) oder nicht reduzierenden (-DTT) -Bedingungen untersucht, und zwar mit dem Fusionskonstrukt TRAILR2:Fc (also einem Konstrukt einer Immunglobulindomäne und eines TNF-Rezeptors) als Kontrolle in den oberhalb von Western-Blot durch eine (+)-Markierung angezeigten Spuren. Links vom Western-Blot sind Marker des Molekulargewichts in kDa angegeben und rechts vom Western-Blot durch Pfeilmarkierung die für Fusionskonstrukt Fc:FasL angezeigten Molekulargewichte (110 kDA bzw. 57 kDa), die in Abhängigkeit von der Ausbildung von Disulfidbrücken (nur unter nicht-reduzierenden Bedingungen) zwischen den Fc-Domänen erwartungsgemäß variieren.

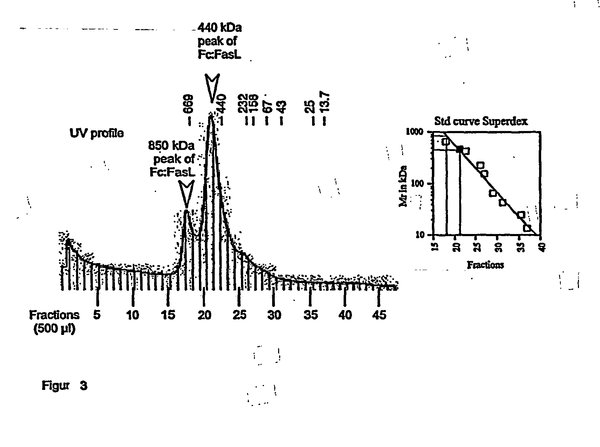

Figur 3 stellt das Ergebnis der Gelpermationschromatographie dar. Hierzu wurde Fc:FasL (300 µg in 200 µl PBS) auf eine Superdex-200 Säule, äquilibriert in PBS, aufgetragen und bei einer Geschwindigkeit von 0,5 ml/min eluiert Das UV-Profil wurde "online" bei 280 nm aufgenommen und 0,5 ml Fraktionen wurden jeweils gesammelt. Die Elutioasposition ist unterhalb von Figur 3 aufgetragen und das Molekulargewicht (m kDa) sowie die Kalibrationsstandards sind oberhalb vom Profil aufgetragen. Das apparente Molekulargewicht der beiden Peaks von Fc:FasL wurde aus der Kalibrationskurve, die in der rechten Darstellung von Figur 3 gezeigt ist, abgeleitet Das Ergebnis ist oberhalb der beiden Peaks (850 kDa bzw. 440kDa) dargestellt Unterhalb vom Profil sind die Maßzahlen der untersuchten Fraktionen in der Reihenfolge der Elution dargestellt

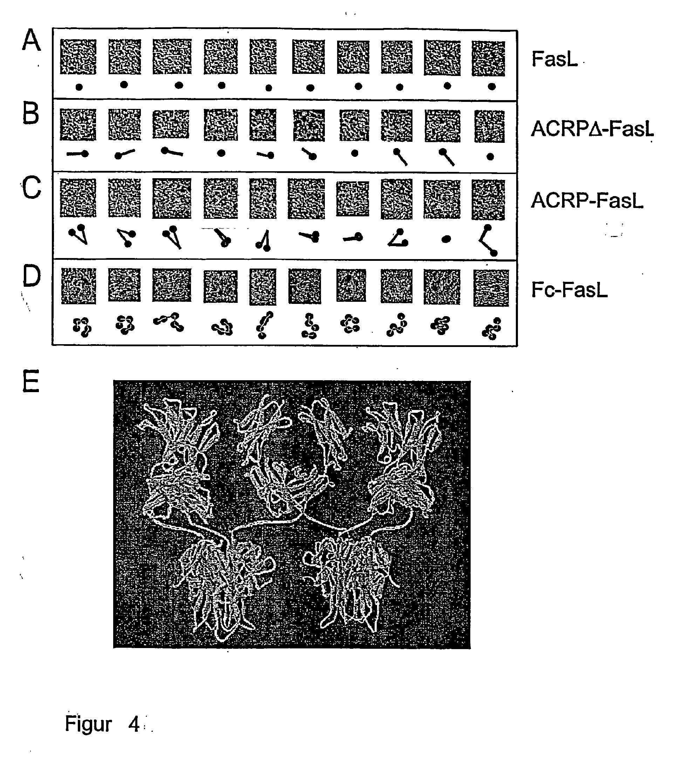

In Figur 4 sind elektronenmikroskopische Aufnahmen dargestellt, und zwar in der Reihenfolge der Teilfiguren von FasL (Figur 4a), von ACRPΔ-FasL (Figur 4b), ACRP-FasL (Figur 4c) und schließlich Fc-FasL (Figur 4d). Die einzelnen Teilfiguren enthalten jeweils verschiedene Präparationen der injizierten Aminosäuresequenzen in alternativen Aufsichten, wobei unterhalb der Aufnahmen eine schematische Interpretation des elektronenmikroskopisch bestimmten Bildes dargestellt ist Hierbei ergibt sich aus den elektronenmikroskopischen Aufnahmen gemäß Figur 4a ein Punktmodell für FasL (3 ch, also der erwartete Trimer von multimetisierendem FasL), für ACRPΔ:FasL (Figur 4b), ein stäbchenförmiges Modell mit Punktende, wobei der Punkt den FasL-Bestandteil markiert und die Stäbchenform die Kollagendomäne von ACRPΔ. Gemäß Figur 4c ergibt sich für ACRP:FasL eine zwillingskirschenartige Struktur zweier verbundener Stäbchen (ACRP30-Kollagendomäne mit angefügten FasL-Domäne). Schließlich ist gemäß Figur 4d die Struktur von Fc:FasL erkennbar, die hellen Punkte stehen für FasL, die dunklen Punkte für den Fc-Anteil von IgG1. Die verbindende Weißlinie gibt die Konnektivität zwischen den verschiedenen Untereinheiten wieder.
Figur 4e ist eine modellhafte Darstellung der dreidimensionalen Struktur eines Fc:Liganden-Komplexes. Als Ligand wurde die Struktur von Lymphotoxin A (PDB-Zugangsnummer 1TNR) und als Fc-Komponente IgG 2a (PDB-Zugangsnummer 1IGT) gewählt

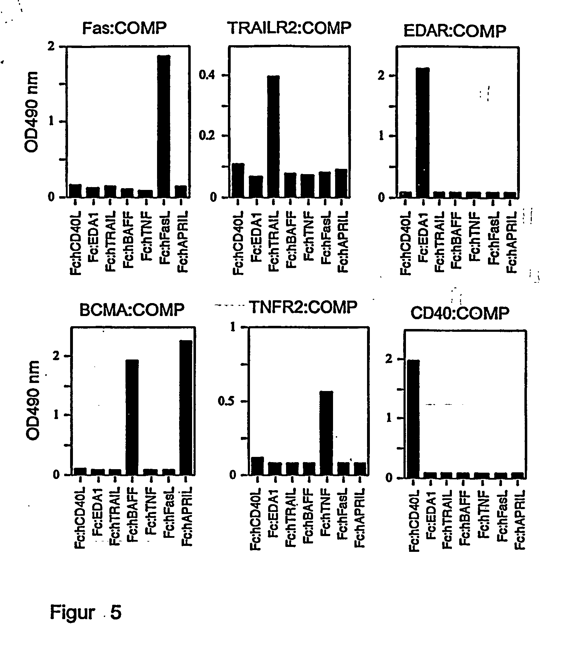

Figur 5 zeigt die Spezifizität der Bindung von Fc-Liganden an ihre jeweiligen Rezeptoren. Hierbei wurde die Bindung der Liganden an ihre Rezeptoren (in Gestalt von Rezeptor. COMP-Fusionsprotein) mit Hilfe eines ELISAs gemessen, und zwar unter Verwendung der folgenden Schritte : (a) Beschichtung mit murinem anti-humanen IgG, (b) Hinzufügen der jeweiligen Fc-Liganden, in der gewünschten Konzentration (als Zellüberstände), (c) Hinzufügen des jeweiligen Rezeptor-COMP-Fusionskonstrukts (welche jeweils eine Tag-Markierung tragen und ebenfalls als Zellüberstände hinzugefügt wurden), (d) Hinzufügen von biotinyliertem Anti-Tag-monoklonalem Antikörper, (e) Hinzufügen von Horse Raddish peroxidase (HRP)-gekoppeltem Stteptavidin, (f) Entwicklung des Assays mit OPD-Reagenz und Messung der Absorbanz bei 490 nm.
   Aus den in Figur 5 dargestellten Diagrammen ist deutlich erkennbar, daß eine starke Spezifizität der Fusionskonstrukte für die jeweiligen spezifischen TNF-Rezeptoren der im erfindungsgemäßen Konstrukt enthaltenen Liganden (oder Ligandenabschnitte) besteht, nämlich von FasL für Fas, Trail für Trail R2, EDA1 für den EDA-Rezeptor, erwartungsgemäß BAFF und APRIL für den insoweit bifunktionalen Rezeptor BCMA, TNF für TNFR2 und CD40L für CD40R. Die Darstellung als Konstrukt mit Fc-Anteil hat also keine Auswirkung auf das Bindungsverhalten der Komponente (B) eines Konstrukts an den entsprechenden Rezeptor.

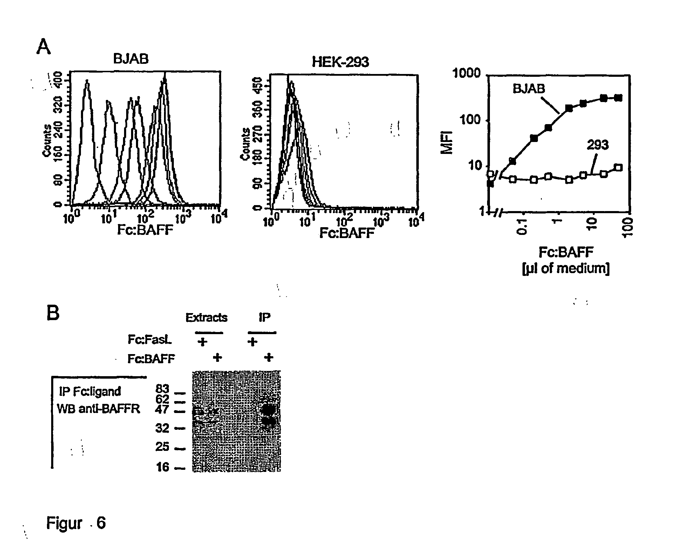

Figur 6 stellt die Ergebnisse von Bindungsexperimenten mit Fc:BAFF dar. Gemäß Figur 6a wurden BJAB-Zellen (BAFF-R positive Zellen und HEK-293-Zellen (BAFF-R negative Zellen) in einem Endvolumen von 100 Miktroliter mit 0,05, 0,2, 0,5, 2,5, 20 oder 50 µl von Zellüberständen, die Fc:BAFF enthielten, inkubiert. Gebundenes Fc:BAFF wurde mit PE-konjugierten antihumanem Ig-Antikötper und FACS-Analyse identifiziert Das Ergebnis einer Quantifizierung der durchschnittlichen Fluoreszenz (MFI) ist in der rechten Darstellung von Figur 6a dargestellt Hierbei zeigt sich nach Auswertung der direkten Auftragungen für BJAB- und HEK 293-Zellen in der Sekundärauftragung (Figur 6a ganz rechts) deutlich, daß die mittlere Fluoreszenz als Maß für die Bindung an die Zellen im Falle von BJAB-Zellen deutlich steigt, während die BAFF-R negativen HEK-293-Zellen auch bei hohen Konzentrationen des erfindungsgemäßen Fc:BAFF keine signifikant erhöhte mittlere Fluoreszenz aufweisen. Die nicht erwartete Bindung von Fc:BAFF an die BAFF-R negativen Zellen findet also im Gegensatz zur Situation bei BAFF-R positiven Zellen auch nicht statt
   Gemäß Figur 6b finden sich die Ergebnisse von Immunopräzipitationsexpetimenten aufgetragen. Hierzu wurden BJAB-Zellen (5x10⁷ pro Ansatz) für 15 Minuten in 2 ml eines Mediums in Gegenwart von 3 µg von Fc:FasL oder von Fc:BAFF inkubiert. Die Zellen wurden geerntet, in PDS gewaschen, in Lysepuffer lysiert (enthaltend 1% NP-40 und mit Protein A-Sepharose immunopräzipitiert. IPs und Gesamtzellextrakte wurden durch Western-Blot-Technik unter Verwendung von murinem anti-hBAFF-R-monoklonalem Antikörper analysiert. Hierbei zeigte sich, daß die BAFF-R-positiven BJAB-Zellen während der Inkubation Fc:BAFF (rechte Spur in Figur 6b) binden und daher im IP-Ansatz durch entsprechende Antikörper nachweisbar sind.

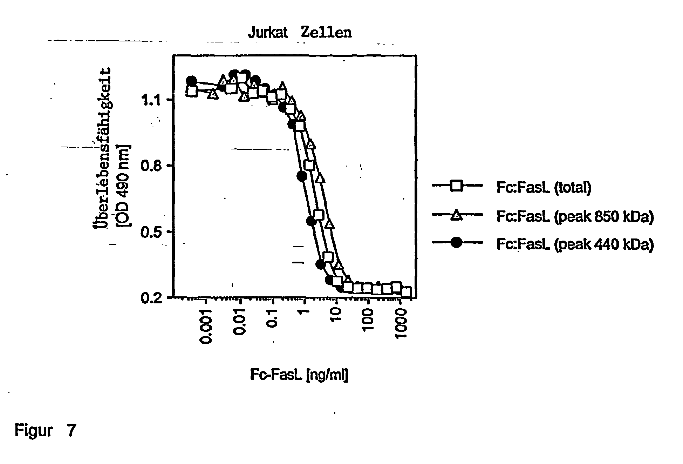

In Figur 7 wird die Cytotoxizität von Fc:FasL dargestellt, und zwar mit Hilfe von Reihenverdünnungen von erfindungsgemäßen Fc:FasL (wahlweise das auf die Säule zugeführte Material, die 850 kDa-Fraktion und die 440 kDa-Fraktion, wie gemäß Figur 3 dargestellt). Diese Reihenverdünnungen wurden auf FasL-sensitive Jurkat-Zellen gegeben und für die Dauer von 16 Stunden bei 37° inkubiert Die Lebensfähigkeit der Zellen wurde danach mit einem PMS/MTS-Assay beobachtet. Hierbei zeigte sich, daß die Gesamtfraktion ebenso wie die Einzelfraktionen ein sehr ähnliches Profil im Hinblick auf die Überlebensfähigkeit der Zellen aufweisen.

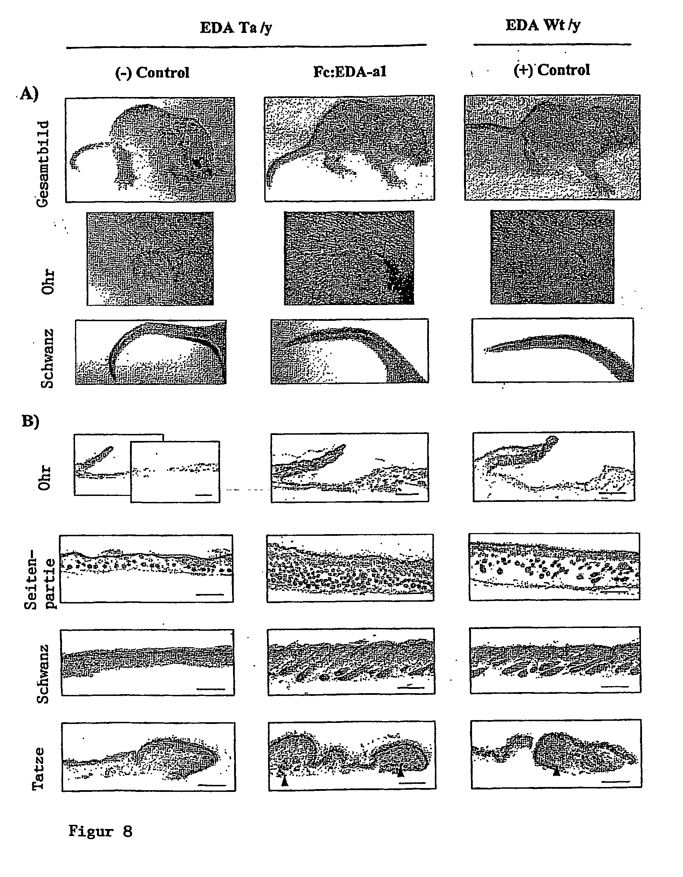

Figur 8 zeigt die phänotypischen Unterschiede zwischen erfindungsgemäß mit Fc:EDA1 (s. Fig. 1d) behandelten oder unbehandelten Tabby-Mäusen 10 Tage nach der Geburt. Als Kontrolle dienten WT-Mäuse (rechts). Hierzu wurde trächtigen Tabby-Mäusen intravenös 400 µg von Fc:EDA1 am Tag 11, 13, und 15 der Trächtigkeit injiziert. Nachkommen von behandelten Tabby-Mäusen, nicht-behandelten Tabby-Mäusen und Wildtypmäusen wurden untersucht. Hierbei stellt gemäß Figur 8a auf das Erscheinungsbild der Ohrpartie, insbesondere auf den Haarwuchs der Ohrpartie ab. Der Haarwuchs bei behandelten Tieren entspricht dem Haarwuchs bei WT-Tieren. Auch die Schwanzpartie bei behandelten Mäusen weist wie bei WT-Mäusen sowohl eine entsprechende Behaarung als auch die gewohnte Form auf. Der Unterschied zu nicht-behandelten Mäusen ist direkt offensichtlich.
   Gemäß Figur 8b sind histologische Gewebeschnittdarstellungen der retroorikularen Region, der Körperseite, des Schwanzes und der Tatze in Paraffin unter Verwendung von Hämatoxylin-Eosinfärbung. Unter dem Ohr von unbehandelten Tabby-Mäusen ist eine ausgeprägte kahle Region zu erkennen. Behandelte Tabby-Mäuse gleichen insoweit phänotypisch dem WT. Die Körperseite von behandelten Tabby-Mäusen zeigt eine WTartige Vergrößerung der Zahl der Haarfollikel gegenüber unbehandelten Tabby-Mäusen. Im Schwanzbereich weisen die behandelten Mäuse den Haarwuchs von WT-Mäusen auf. Nicht-behandelte Tabby-Mäuse sind dagegen im Schwanzbereich haarlos. Im Gewebe der Tatze von behandelten Mäusen (Mitte) sind, wie durch die Pfeile angedeutet, Schweißdrüsen zu erkennen, wie auch im WT. Nicht behandelte Tabby-Mäuse entwickeln demgegenüber keine Schweißdrüsen. Die folgenden Merkmale wurden verglichen:
   Gemäß Figur 9 sind vergleichend die Ergebnisse einer Behandlung von Tabby-Mäusen in utero mit dem erfindungsgemäßen Konstrukt gemäß Figur 1d und von entsprechenden Kontrolltieren.
Figur 9a zeigt einen Vergleich von behandelten Tabby-Mäusen (Mitte) und nicht behandelten Tabby-Mäusen (links) im ausgewachsenen Alter (6 Wochen nach Geburt). Die Behandlung erfolgte so wie gemäß Ausführungsbeispiel 4 dargestellt Rechts ist das Erscheinungsbild von gesunden Mäusen als Kontrolle festgehalten. Die behandelten Tiere besitzen ein Fell wie die WT-Tiere (auch in dem kritischen Abschnitt am Schwanz), der Schwanz der behandelten Mäuse zeigt keine gezacktes Erscheinungsbild, die Haare von behandelten Mäusen zeigen insbesondere den für WT-Mäuse typischen Haartyp "monotrisch", vor allem eine langes monotrisches Deckhaar, der bei den unbehandelten Tabby-Mäusen vollständig fehlt.
Figur 9b vergleicht die Kiefer- und Zahnstruktur von behandelten, unbehandelten Tabby-Mäusen und WT-Mäusen. Die unbehandelten Tabby-Mäuse weisen eine abnorme Kieferstruktur auf und auch die Zähne zeigen Deformitäten, insbesondere wenig ausgeprägte Zahnhöcker. Die Behandlung durch ein erfindungsgemäßes Verfahren während der Trächtigkeit (Behandlung des Muttertieres) hat die natürliche Zahnform wiederherstellen können.
Figur 9c zeigt Gewebeschnitte des Augenlids und der Komea bei unbehandelten, erfindungsgemäß mit Fc:EDA1 behandelten Tabby-Mäusen und WT-Kontrolltieren. Bei den unbehandelten Tabby-Mäusen fehlen charakteristischer Weise die Moebius-Drüsen am Augenlid. Diese Drüsen treten jedoch nach erfindungsgemäßer Behandlung wieder auf. Durch die Regenerierung der Moebius-Drüsen wird auch die Keratinisierung der Kornea vermieden, wie sie, aus dem Gewebeschnitt gemäß Figur 9c deutlich ersichtlich, bei unbehandelten Tabby-Mäusen auftritt.
In Figur 9d sind die Ergebnisse des Schweißtests dargestellt, die zeigen, dass bei erfindungsgemäß behandelten Tabby-Mäusen funktionale Schweißdrüsen an der Tatze beobachtet werden können, die bei unbehandelten Tabby-Mäusen grundsätzlich fehlen. Derartige wiedergewonnene Schweißdrüsen nach Behandlung mit Fc:EDA1 ermöglichen es die typischen physiologischen Reaktionen auf Stimuli, wie etwa Hitze und Streß, zu zeigen. Der Schweißtest selbst ist in Ausführungsbeispiel 4 beschrieben.

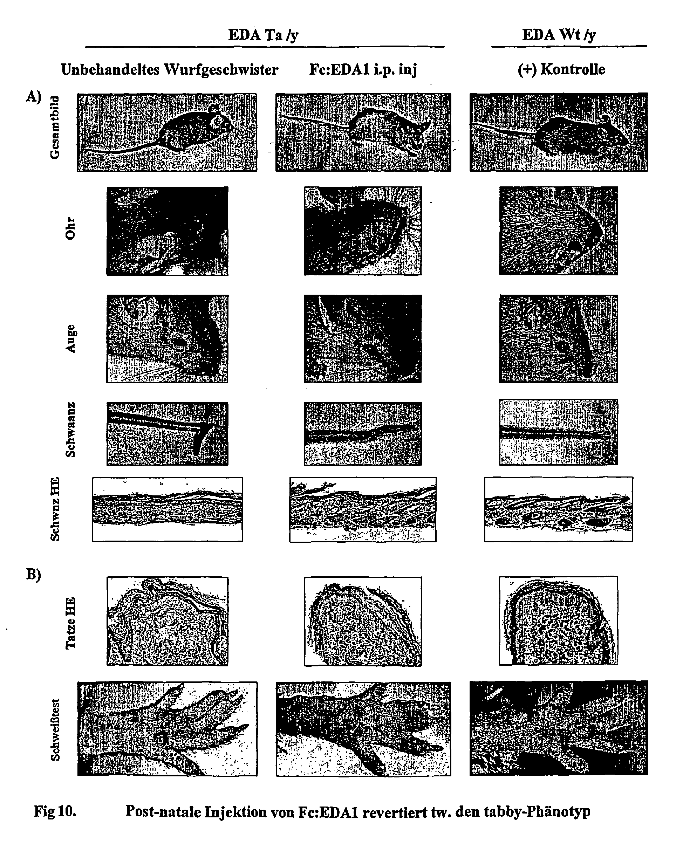

Figur 10 stellt die Ergebnisse postnataler intraperitonealer Behandlung mit erfindungsgemäßem Fc:EDA1-Fusionskonstrukt der Tabby-Mäuse im Vergleich zu unbehandelten Tabby-Mäusen und WT-Kontrolltieren dar.
Figur 10a vergleicht verschiedene Körperpartien in phänotypischer Hinsicht bei den vorgenannten drei Tiergruppen. Der Hautbereich hinter den Ohren wies auch bei adulten, postnatal behandelten Tieren, im Gegensatz zum WT, keine Behaarung auf. Hingegen konnten durch postnatale Injektion von Fc:EDA1 einerseits die Schwanzbehaarung wie beim WT und andererseits die für den WT typische Größe der Augenöffnung nahezu wiederhergestellt werden. Die "geknickte" Schwanzform konnte durch die erfindungsgemäße postnatale Behandlung nahezu beseitigt werden. Darüber hinaus zeigen Gewebeschnitte des Schwanzes, die mit Hämatoxillin und Eosin angefärbt wurden, sowohl zahlreiche Haarfollikel als auch mit den Haarfollikeln assoziierte Talgdrüsen.
Figur 10b stellt mikroskopisch und makroskopisch die Phänotypen der drei Gruppen in Hinblick auf die Schweißdrüsen an den Fußtatzen dar. Eine Vielzahl von Schweißdrüsen an den Tatzen wurde nach der Behandlung bei postnatal behandelten adulten Tabby-Tieren beobachtet, die von den Schweißdrüsen der WT ununterscheidbar waren, wie in Figur 10b anhand von mit Hämatoxillin und Eosin angefärbten Gewebeschnitten gezeigt. Diese Schweißdrüsen bei behandelten Tabby-Tieren erwiesen sich einem entsprechenden Schweißtest als funktional und reagierten auf physiologische Stimuli, wie bspw. Hitze oder Streß.

Zusammenfassen ist also festzustellen, dass die - im Falle einer Behandlung in utero - nach der Geburt beobachtete Reversion des Phänotyps beim adulten Tier nicht mehr verschwindet, ebenso wie auch die sich bei postnataler Behandlung mit erfindungsgemäßen Substanzen einstellenden phänotypischen Reversionen beim Jungtier gleichfalls auch beim ausgewachsenen Tier bestehen bleiben.

Die vorliegende Erfindung wird durch die nachfolgenden Ausführungsbeispiele näher erläutert.

### Ausführungsbeispiele

### 1. Ausführungsbeispiel

### Herstellung von Fc: Liganden-Fusionsproteinen

Bevorzugte erfindungsgemäße Fusionsproteine bestehen aus einem Fc-Anteil von humanem Immunglobulin (Komponente (A)), wobei die zugrundeliegende DNA-Sequenz das Stopcodon des Immunglobulin-Fragments nicht enthält, und einem C-terminalen Abschnitt eines Fragments eines Liganden der TNF-Familie (Komponente (B)) (mit Stop-Codon der zugrundliegenden DNA-Sequenz). Hierbei weisen bevorzugte erfindungsgemäße Fusionsproteine die folgende Struktur auf: am N-Terminus ein Signalpeptid, dann einen Fc-Abschnitt bspw. von IgG, einen Übergangsbereich, enthaltend einen Linkerbereich und eine Protease-Schnittstelle und einen C-terminalen Abschnitt des TNF-Liganden EDA1. Eine schematische Darstellung eines bevorzugten erfindungsgemäßen Fusionsproteins ist in Figur 1d dargestellt.

Zur Herstellung eines erfindungsgemäßen Fusionsproteins, bspw. eines Fusionsproteins mit einer bevorzugten Struktur gemäß Fig. 1d, wurden Nukleinsäurekonstrukte in Expressionsvektoren von Säugetieren kloniert und in stabilen humanen embryonalen Nierenzellinien (in HEK-293 oder in CHO (Chinese hamster ovary)-Zellen) exprimiert. Die sekretierten rekombinanten Fusionsproteine wurden aus dem konditionierten Medium durch Affinitätschromatographie auf einer Protein-A-Sepharose-Säule gewonnen. Die Ausbeuten betrugen bis zu mehreren mg pro Liter des eingesetzten Mediums.

### 2. Ausführungsbeispiel

### Biochemische Charakterisierung von Fc:FasL

Die biochemische Charakterisierung von FasL erfolgt durch SDS-PAGE-Analyse, wobei ein apparentes Molekulargewicht von 57 kDa bzw. ungefähr 110 kDa unter reduzierenden bzw. nicht reduzierenden Bedingungen ermittelt wurde. Hierdurch wurde die Gegenwart von über Disulfidbrücken verbundenen Dimeren (2 ch) nachgewiesen, wie es von Fc-enthaltenden Fusionsproteinen mit "Hinge-Region" zu erwarten war (siehe auch Fig. 2).
Eine entsprechenden Untersuchung durch Gelpermationschromatographie zeigte, daß zwei gut definierte "Peaks" von Fc:FasL mit apparentem Molekulargewicht von ungefähr 440 kDa (Hauptpeak) bzw. ungefähr 850 kDa (Nebenpeak) eluierten (siehe Fig. 3). Unter der Annahme einer globulären Gestalt von FcFasL betrug die berechnete Multimer-Struktur von FasL 7,7 (7,7 ch) für die Hauptspezies im Elutionsprofil. Im Falle eines nicht idealtypisch globulären Proteins beruht diese Berechnung jedoch mit hoher Wahrscheinlichkeit auf einer Überschätzung des Molekulargewichts, so daß eine dimere Ligandenstruktur (6 ch) durchaus möglich wäre. Die dem 440 kDa Peak entsprechende Form von Fc:FasL wurde mit Hilfe der Elektronenmikroskopie untersucht, wobei zum Vergleich FasL (3 ch), oligomeres ACRP30:FasL (6+ ch) und eine Deletionsmutante von ACRP30:FasL, nämlich ACRPΔ:FasL (3 ch) herangezogen wurden. FasL (3 ch) weist eine ballähnliche Gestalt auf (siehe Fig. 4a), ACRPΔ:FasL (3 ch) hat eine ballähnliche Gestalt mit einem "Griff" (siehe Fig. 4b) und ACRP30: FasL sieht aus wie eine "Zwillingskirscht", was im Ergebnis mit einer dimeren FasL (6 ch)-Struktur (siehe Fig. 4c) übereinstimmt.

Fc:FasL nimmt regelmäßig eine Ringstruktur mit 5 "Bällen" oder eine offene Struktur mit 5 "Bällen" an (siehe Figur 4d). Dies wiederum entspricht einem dimeren FasL (6 ch), wobei die beiden Bälle FasL und die drei übrigen Bälle die Fc-Bestandteile von IgG (3 Polypeptiddimere: 6 ch) sind. Eine modellhafte Darstellung eines Fc-Liganden wurde unter Verwendung der bekannten Kristallstruktur von Lymphotoxin a (ein Mitglied der TNF-Familie) und des Fc-Bestandteils von IgG2a (PDB-Zugangsnummem 1TNR und 1IGT) entwickelt, wobei der Linker-Bereich als durchgezogene Linie eingezeichnet ist (siehe Figur 4e). Hierdurch wird eindeutig gezeigt, daß die Liganden und die Fe-Anteile ungefähr ähnliche Größen besitzen, in Übereinstimmung mit der beobachteten 5-Ballstruktut von Fc:FasL. Alles in allem zeigen diese Ergebnisse an, daß der 440 kDa Peak von Fc:FasL ein dimeres FasL (6 ch) ist

Die Bezeichnung "ch" steht in der vorliegenden Offenbarung für "chain" und spiegelt damit die Zahl der Ketten des TNF-Liganden (bspw. APRIL, FasL, BAFF, EDA oder Tweak) im Molekül wider (unabhängig davon, ob ein Fusionskonstrukt betrachtet wird oder nicht). Liegt ein TNF-Ligand in trimerer Form vor, hat er drei Ketten (3 ch), ein Dimer eines Liganden (bspw. über die erfindungsgemäße Fc-Konstruktion) hat 6 Ligandenketten (6 ch),

Analog erfolgte auch die Charakterisierung der übrigen in den Figuren 1b bis 1j dargestellten erfindungsgemäßen Fusionsprotein-Sequenzen.

### 3. Ausführungsbeispiel

### Biologische Aktivität der Fc: Liganden-Konstrukte in vitro:

Erfindungsgemäß wurde ein ELISA-basierter Assay entwickelt, um die biologischen Eigenschaften von Fc:Liganden-Konstrukten, nämlich an ihre entsprechenden Rezeptoren zu binden, zu untersuchen. In diesem Assay wurden die FcLiganden-Konstrukte zunächst mit einem murinen antihumanen IgG monoklonalen Antikörper gefangen. In einem zweiten Schritt wurden die löslichen Rezeptoren an die Oligomerisierungsdomäne des "cartilage oligomeric matrix protein (COMP) (s. DE 19963859 und DE 10122140, die jeweils vollinhaltlich Bestandteil der vorliegenden Offenbarung sind) fusioniert und eine Flag-Sequenz wurde hinzugefügt. Rezeptoren mit gebundenen Liganden wurde mit Hilfe ihrer jeweiligen Flagtags identifiziert (siehe Figur 5). Durch diese modifizierte Vorgehensweise eines ELISA-Assays wurde gezeigt, daß die erfindungsgemäßen Fc:Liganden-Konstrukte in der Tat an ihre jeweiligen nativen Rezeptoren binden, also ihre Bindungsfähigkeit durch die Konstruktstruktur nicht verlieren, und die Bindung erfolgt auch mit einer hohen Selektivität.

Ferner wurde erfindungsgemäß gezeigt, dass die Fc-Liganden-Konstrukte auch an oberflächenexprimierte, endogene Rezeptoren binden können. Experimentell konnte gezeigt werden, daß Fc:BAFF-Konstrukte BAFF-R-positive BJAB-Zellinien in dosisabhängiger Weise markieren, allerdings nicht die BAFF-R-negative Jurkat-Zellinie (siehe Figur 6a). Zusätzlich konnte gezeigt werden, daß Fc:BAFF den endogenen BAFF-Rezeptor von BJAB-Zellen spezifisch immunopräzipitieren kann (siehe Figur 6b).

Weiterhin wurde die Cytotoxizität von Fc:FasL auf einer FasL-sensitiven Jurkatzellinie untersucht. Fc:FasL erwies sich als hochcytotoxisch auf Jurkatzellen, mit einem IC₅₀ von 1 ng/ml (siehe Figur 7). Zudem wurde festgestellt, daß keine signifikanten Unterschiede in Hinblick auf die spezifische Aktivität zwischen der Gesamtpräparation von Fc:FasL und der im 440 kDa Peak isolierten dimeren Form von Fc:FasL (6 ch) bzw. der im 850 kDa Peak identifizierten oligomeren Form von Fc:FasL (12 ch) besteht Diese Ergebnisse zeigen, daß dimeres FasL (6 ch) bereits ein vollständig aktives Molekül ist und daß größere Komplexe die diesbezügliche Aktivität nicht mehr verstärken.

### 4. Ausführungsbeispiel

### Biologische Aktivität von Fc:Liganden-Konstrukten in vivo:

Es wurden die Fusionsproteine Fc:FasL und Fc:EDA in vivo getestet

Die Injektion von Fc:FasL in Mäuse ergab, daß dieses Fusionsprotein in vivo Cytotoxizität zeigt.
Fusionskonstrukt Fc:EDA: EDA ist ein Mitglied der TNF-Ligandenfamilie, das physiologisch für die Entwicklung und Funktion, insbesondere auch für die Morphogenese von ektodermalen Strukturen verantwortlich ist, ganz besonders für Schweißdrüsen, Haare und Zähne als epitheliale Strukturen des Ektoderms. Umgekehrt ist bekannt, daß Fehlfunktionen von EDA das Krankheitsbild (XL)HED beim Menschen verursacht, während bei Mäusen die Fehlfunktion zu dem sogenannten Tabby-Phänotyp führt, im wesentlichen charakterisiert durch (i) abnorme Struktur des Fells, (ii) Fehlen der unterschiedlichen nativen Haartypen, (iii) Haarverluste am Schwanz und im Hautbereich hinter den Ohren, (iv) Abwesenheit von Schweißdrüsen in den Tatzen, (v) abnorme Zahnbildung und -durchbruch (vi) charakteristische Verdrehung an der Schwanzspitze, (vii) Fehlen von Moebius-(Drüsen im Augenlid, die einen dünnen Fettfilm zum Schutz der Komea vor dem Austrocknen sekretieren; daher Keratinisierung der Cornea) und anderen Drüsen, (viil) verringerte Größe der Augenöffnung und (ix) geringere Gewichtszunahme bei Jungmäusen.

Erfindungsgemäß wurde den weibliche Tabby-Mäusen intravenös 400 µg von Fc:EDA1 am 11., 13. und 15. Tag der Trächtigkeit in utero injiziert. Die Toleranz gegenüber diesem Fusionsprotein war ausgezeichnet und es wurden keine konttaindizierenden Wirkungen bei den behandelten Mäusen beobachtet. Während die Fc:EDA1-Behandlung bei den behandelten weiblichen ausgewachsene Mäusen selbst keine erkennbaren Wirkungen zeigte, wurde eine eindeutige und starke Reversion des Tabby-Phänotyps in der Nachkommenschaft der behandelten trächtigen Muttertiere beobachtet. Diese Reversion des Phänotyps war am 10. Tag nach der Geburt offensichtlich. Die Größe der Nachkommen der während der Trächtigkeit behandelten Tabby-Mäuse war erheblich größer als jene der Kontrollmäuse, ihr Fell war glatt, schwarz und glänzend (siehe Figuren 8/9). Im Gegensatz zu den Tabby-Mäusen erwies sich auch der retroorikulare Bereich der behandelten Tabby-Mäuse als haarig, und sie zeigten normale, ungebogene haarige Schwänze mit zahlreichen Haarfollikeln und den entsprechenden Talgdrüsen, die auf der Haut der Schwanzregion beobachtet werden konnten (siehe Figur 8b).
Darüber hinaus konnten ekkrine Schweißdrüsen, die von den entsprechenden Wildtyp-Schweißdrüsen nicht unterscheidbar waren, auf den Fußtatzen von behandelten, nicht aber von unbehandelten Täbby-Mäusen im Gewebeschnitt erkannt werden (siehe Figur 8b). Diese Schweißdrüsen waren auch bei den in utero im embryonalen Stadium behandelten Tabby-Mäusen noch im Erwachsenenalter funktional. Zur Ermittlung der Funktionalität der Drüsen wurde ein Schweißtest durchgeführt: Hierzu wurden die Tatzen der untersuchten Tieren mit 3,5% (w/v) I₂-Lösung in Ethanol eingestrichen, dann der Trocknung überlassen. Anschließend wurde eine 50% (w/v) Stärkelösung in Mineralöl aufgetragen. Nach Anwendung mäßiger Hitze mit einem Fön (5 s) wurde eine Aufnahme gemacht Tiere wurden geopfert und Hämatoxilin und Eosin-gefärbte Gewebeschnitte des Augenlids und der Comea hergestellt.

Der revertierte Phänotyp war stabil, wie durch die Analyse von behandelten Tabby-Mäusen im ausgewachsenen Zustand bestätigt werden konnte (s. Fig. 9). Sie waren signifikant größer als Tabby-Mäuse und ihr Fell zeigte insbesondere die längere Wildtyp-Haarart monotrisch, die bei den Tabby-Mäusen vollständig fehlt Sie hatten keine kahlen Stellen in der retroorikularen Region, die Fußtatzen wiesen ekkrine Schweißdrüsen auf, nicht unterscheidbar vom Wildtyp. Diese Schweißdrüsen waren darüber hinaus funktionell (Figur 9a), wie im vorbeschriebenen Schweißtest nachgewiesen. Die Moebius-Drüsen, die in Tabby-Mäusen stets fehlen, waren nach Behandlung wieder vorhanden und ließen die Bildung normaler, nicht-keratinisierter Cornea zu, was die Funktionstüchtigkeit der Moebius-Drüsen beweist (Fig. 9c). Die Zähne von behandelten Tabby-Mäusen hatten eine normale Größe und zeigten ein Wildtypmuster von Zahnhöckern. Der dritte Molar ist in Wildtypmäusen klein und in Tabby-Mäusen nicht vorhanden. Dieser dritte Molar fehlte auch in behandelten Tabby-Mäusen. Die Ergebnisse zeigen, daß erfindungsgemäßes Fc:EDA1 gemäß Fig. 1d, trächtigen Mäusen verabreicht, in den sich entwickelnden Embryo gelangen kann, und zwar via Fc-Rezeptoren der Placenta und an EDA-Rezeptoren die Induktion der Bildung von nativen epithelialen Strukturen auslösen kann. Diesbezüglich ist die Dimerisierung von EDA1 (6 ch) mit hoher Wahrscheinlichkeit von zentraler Bedeutung, da genetische und biochemische Untersuchungen anzeigen, daß die Oligomerisierung von EDA für dessen Aktivität essentiell ist. Weiterhin wurde erfindungsgemäß gezeigt, daß funktionelles EDA nach Induktion und Etablierung epithelialer Strukturen im sich entwickelnden Embryo für die Aufrechterhaltung des revertierten nativen Phänotyps dem erwachsenen Tier nicht mehr zugefügt werden muß.

### 5. Ausführungsbeispiel

In diesem Beispiel wurde untersucht, inwieweit Symptome des Tabby-Phänotyps der Maus durch eine späte, postnatale Verabreichung von erfindungsgemäßem Fc:EDA1 aufgehoben werden können. Daher wurde Fc:EDA1 am 2. (40 µg), 4. (40 µg), 6. (60 µg), 8. (100 µg) und 10. (100 µg) Tag (oder in einem alternativen Versuchsansatz: am 5. (40 µg), am 7. (60 µg), am 9. (60 µg), am 11 und am 13. (jeweils 100 µg) Tag nach der Geburt) intraperitoneal den Jungmäusen verabreicht bzw. keine Behandlung in einer Kontrollgruppe von Tabby-Mäusen vorgenommen.

Die entsprechend behandelten Mäuse oder Mäuse der Kontrollgruppe wurden auf die folgenden phänotypischen Merkmale hin untersucht: (i) "geknickter" Schwanz, (ii) Haarbildung am Schwanz bzw. hinter dem Ohrenbereich, (iii) Augenform. Weiterhin wurden bei adulten Mäusen ein Schweißtest durchgeführt und bei 25 Tage alten Mäusen wurden Gewebeschnitte aus der Tatze und Hautbereichen des Schwanzes untersucht

Auch bei dieser postnatalen Verabreichung von Fc:EDA1 konnten zahlreiche Symptome des Tabby-Phänotyps, insbesondere die Wiederherstellung des Schwanzhaares und der Schweißdrüsen, herbeigeführt werden. Das Schwanzhaar konnte am 15. Tag bei den (am 2. Tag nach der Geburt bereits) Fc:EDA1-behandelten Tabby-Mäusen beobachtet werden (anstatt am 7. bis 9. Tag nach Geburt bei WT-Mäusen). Bei den erst am 5. Tag nach der Geburt behandelten Mäusen wurde die Haarbildung am 18. Tag beobachtet. Die Haarbildung am Schwanz tritt bei den behandelten Tabby-Mäusen zunächst auf der ventralen Seite des Schwanzes auf. Bei allen behandelten Tabby-Mäusen, unabhängig vom Zeitpunkt des Behandlungsbeginns, unterschied sich das Schwanzhaar bezüglich Struktur und Dichte nicht vom Schwanzhaar bei WT-Tieren. Diese verspätete Haarbildung bei den behandelten Tabby-Mäusen kann ohne weiteres durch die spätere Induktion der Haarfollikelbildung, ausgelöst durch das verabreichte erfindungsgemäße Fc:EDA1, erklärt werden. Die Haarlosigkeit (Alopezie) hinter den Ohren konnte bei den in diesem Ausführungsbeispiel beschriebenen Versuchsansätzen nicht aufgehoben werden.

Die geknickte Schwanzform konnte zwar nicht vollständig, jedoch im wesentlichen durch die postnatale Therapie rückgebildet werden. Die Augenöffnung der behandelten Tabby-Mäuse war gegenüber nicht-behandelten signifikant vergrößert, allerdings nicht genauso groß wie beim WT (Figur 10a). Funktionale Schweißdrüsen entwickelten sich in den Tatzen von behandelten Tabby-Mäusen, wie in Gewebeschnitten bei 25 Tage alten behandelten Tabby-Mäusen nachgewiesen werden konnte (Fig. 10b). In Schweißtests bei adulten behandelten Tabby-Tieren konnte die Funktionalität der durch die Behandlung entwickelten Schweißdrüsen bei erwachsenen Tieren gezeigt werden.

Zusammengefaßt läßt sich feststellen, daß die erfindungsgemäße Experimente eine quasi vollständige Reversion eines Phänotyps, der genetisch auf die Absenz des TNF-Liganden EDA zurückzuführen ist, hervorrufen kann. Die besten Heilungserfolge konnten bei den bereits in utero mit erfindungsgemäßem rekombinantem Fusionsprotein behandelten Tabby-Mäusen beobachtet werden. Allerdings führt auch eine postnatale Verabreichung erfindungsgemäßer Fusionsproteine, bspw. Fc:EDA1, bei genotypisch betroffenen Tieren zu einem beachtlichen Heilungserfolg, d.h. zu einer (Teil)reversion des Phänotyps, insbesondere zur Entwicklung von Schweißdrüsen (vor allem in den Tatzen), Schwanzhaar und zu einer Vergrößerung der Augenöffnung.

## Patentansprüche

1. Rekombinantes Fusionsprotein, enthaltend eine Aminosäuresequenz, die umfaßt: (a) im N-terminal gelegenen Abschnitt des Fusionsproteins, den Fc-Abschnitt eines Immunoglobulins oder einen Teil eines Fc-Abschnitts eines Immunglobulins der die Fähigkeit zur Dimerisierung beibehält als Komponente (A) , (b) im weiter C-terminal gelegenen Abschnitt des Fusionsproteins, den extrazellulären Teil eines TNF-Liganden oder eine Teilsequenz des extrazellulären Teil eines TNF-Liganden als Komponente (B) und gegebenfalls (c) einen Übergangsbereich zwischen Komponente (A) und Komponente (B), enthaltend einen Linker,
**dadurch gekennzeichnet, daß** die Komponente (B) der extrazelluläre Teil des TNF-Liganden EDA1 ist oder eine Teilsequenz des extrazellulären Teil des TNF-Liganden EDA1 ist, die mindestens die Aminosäurensequenz 246 bis 391 der nativen Sequenz des EDA1 aufweist.

2. Rekombinantes Fusionsprotein nach Anspruch 1, **dadurch gekennzeichnet, daß** die Komponente (B) die Aminosäuresequenz 140-391, 157-391, 160-391, 181-391, 182-391, 200-391 oder 245-391 der nativen Sequenz des EDA1 aufweist.

3. Rekombinantes Fusionsprotein nach Anspruch 1 oder 2, **dadurch gekennzeichnet, daß** die Komponente (A) die "Hinge"-Region, die CH2 und die CH3-Domäne des Fc-Abschnitts eines Immunglobulins aus der Klasse der IgG, insbesondere von humanem IgG, aufweist.

4. Rekombinantes Fusionsprotein nach einem der vorgenannten Ansprüche, **dadurch gekennzeichnet, daß** die Komponente (A) die Aminosäuresequenz aufweist.

5. Rekombinantes Fusionsprotein nach einem der vorgenannten Ansprüche, **dadurch gekennzeichnet, daß** der Übergangsbereich zwischen Komponente (A) und Komponente (B) die Sequenz PQPQPKPQPKPEPE enthält.

6. Rekombinantes Fusionsprotein nach einem der vorgenannten Ansprüche, **dadurch gekennzeichnet, daß** der Übergangsbereich eine Protease-Schnittstelle aufweist.

7. Rekombinantes Fusionsprotein nach einem der vorgenannten Ansprüche, **dadurch gekennzeichnet, daß** der N-Terminus des rekombinanten Fusionsproteins eine Signalsequenz, beispielsweise eine Sekretionssignalsequenz, und/oder eine Tag-Markierung beispielsweise Flag- oder His-Tag, aufweist.

8. Rekombinantes Fusionsprotein nach einem der vorgenannten Ansprüche, **dadurch gekennzeichnet, daß** der N-Terminus die Sequenz MAIIYLILLFTAVRG aufweist.

9. Rekombinantes Fusionsprotein nach einem der vorgenannten Ansprüche, **dadurch gekennzeichnet, daß** durch die Sequenz des rekombinanten Fusionsproteins funktionell 6 Komponenten (B) miteinander verbunden werden.

10. Komplex aus Fusionskonstrukten nach einem der Ansprüche 1 bis 9, **dadurch gekennzeichnet, daß** der Komplex 6 Ketten der Komponente (A) aufweist.

11. DNA-Sequenz, **dadurch gekennzeichnet, daß** die DNA-Sequenz für ein rekombinantes Fusionsprotein nach einem der Ansprüche 1 bis 9 codiert.

12. Expressionsvektor, **dadurch gekennzeichnet, daß** der Expressionsvektor eine DNA-Sequenz nach Anspruch 11 enthält.

13. Wirtszelle, **dadurch gekennzeichnet, daß** die Wirtszelle mit einem Expressionsvektor nach Anspruch 12 transfiziert ist.

14. Arzneimittel, enthaltend ein rekombinantes Fusionsprotein nach einem der Ansprüche 1 bis 9, ein Komplex nach Anspruch 10, eine DNA-Sequenz nach Anspruch 11, einen Expressionsvektor nach Anspruch 12 oder eine Wirtszelle nach Anspruch 13.

15. Verwendung eines rekombinanten Fusionsproteins nach einem der Ansprüche 1 bis 9, eines Komplexes nach Anspruch 10, einer DNA-Sequenz nach Anspruch 11, eines Expressionsvektors nach Anspruch 12 oder einer Wirtszelle nach Anspruch 13 zur Herstellung eines Arzneimittels für die Behandlung von genetischen Erkrankungen, insbesondere einer ektodermalen Dysplasie, oder Erkrankungen, Störungen oder Anomalien ektodermaler Strukturen, wie beispielsweise Anomalien des Haares, der Zähne oder der Drüsen, insbesondere zur Behandlung von Alopezie oder zur Wundheilung.

16. Verwendung nach Anspruch 15 zur Herstellung eines Arzneimittels zur Behandlung von ektodermaler Dysplasie, insbesondere X-gekoppelter hypohydrotischer ektodermaler Dysplasie.

17. Verwendung nach Anspruch 15 zur Herstellung eines Arzneimittels zur Behandlung von Alopezie, Hirsutismus, zur Behandlung von Wunden oder Schweißbeziehungsweise Talgdrüsenfehlfunktion beziehungsweise einer Defizienz von Schweiß- oder Talgdrüsen.

18. Verwendung nach einem der Ansprüche 15-17 zur Herstellung eines Arzneimittels zur Behandlung der Mutter/dem Muttertier während der Schwangerschaft/Trächtigkeit wobei das zur Verfügung gestellte Arzneimittel parenteral in entsprechender Dosierung verabreicht wird.

## Claims

1. A recombinant fusion protein, containing an amino acid sequence, which comprises: (a) in the section located in the N-terminal of the fusion protein, the Fc section of an immunoglobulin or a portion of an Fc section of an immunoglobulin which retains the capability of dimerization, as a component A), b) in the section further located in the N-terminal of the fusion protein, the extracellular portion of a TNF ligand or a partial sequence of the extracellular portion of a TNF ligand as a component (B) and optionally (c) a transition region between component (A) and component (B), containing a linker,
**characterized in that** the component (B) is the extracellular portion of the TNF ligand EDA1 or is a partial sequence of the extracellular portion of the TNF ligand EDA1, which has at least the 246 to 391 amino sequence of the native sequence of EDA1.

2. The recombinant fusion protein according to claim 1, **characterized in that** the component (B) has the 140-391, 157-391, 160-391, 181-391, 182-391, 200-391 or 245-391 amino sequence of the native sequence of EDA1.

3. The recombinant fusion protein according to claim 1 or 2, **characterized in that** the component (A) has the "hinge" region, the CH2 and CH3 domain of the Fc section of an immunoglobulin of the IgG class, in particular of human IgG.

4. The recombinant fusion protein according to any of the preceding claims, **characterized in that** the component (A) has the amino acid sequence

5. The recombinant fusion protein according to any of the preceding claims, **characterized in that** the transition region between component (A) and component (B) has the sequence PQPQPKPQPKPEPE.

6. The recombinant fusion protein according to any of the preceding claims, **characterized in that** the transition region has a protease restriction site.

7. The recombinant fusion protein according to any of the preceding claims, **characterized in that** the N-terminus of the recombinant fusion protein has a signal sequence, for example a secretion signal sequence, and/or a tag label for example a flag-tag or his-tag.

8. The recombinant fusion protein according to any of the preceding claims, **characterized in that** the N-terminus has the sequence MAIIYLILLFTAVRG.

9. The recombinant fusion protein according to any of the preceding claims, **characterized in that**, 6 components (B) are functionally, bound together by the sequence of the recombinant fusion protein.

10. A complex of fusion proteins according to any of claims 1 to 9, **characterized in that** the complex has 6 chains of the component (A).

11. A DNA sequence, **characterized in that** the DNA sequence codes for a recombinant fusion protein according to any of claims 1 to 9.

12. An expression vector, **characterized in that** the expression vector contains a DNA sequence according to claim 11.

13. A host cell, **characterized in that** the host cell is transfected with an expression vector according to claim 12.

14. A drug, containing a recombinant fusion protein according to any of claims 1 to 9, a complex according to claim 10, a DNA sequence according to claim 11, an expression vector according to claim 12 or a host cell according to claim 13.

15. The use of a recombinant fusion protein according to any of claims 1 to 9, a complex according to claim 10, a DNA sequence according to claim 11, an expression vector according to claim 12 or a host cell according to claim 14, for preparing a drug for treating genetic diseases, in particular ectodermal dysplasia, or either diseases, disorders or abnormalities of ectodermal structures, such as for example abnormalities of hair, teeth or glands, in particular for treating alopecia or for healing wounds.

16. The use according to claim 15 for preparing a drug for treating ectodermal dysplasia, in particular X-coupled hypohydrotic ectodermal dysplasia.

17. The use according to claim 15 for preparing a drug for treating alopecia, hirsutism, for treating wounds or malfunction of sudoral or sebaceous glands, or rather deficiency of sudoral or sebaceous glands.

18. The use according to any of claims 15 to 17 for preparing a drug for treating the mother/the mother animal during pregnancy/gestation wherein the available drug is administered in a suitable dosage parenterally.

## Revendications

1. Protéine de fusion recombinante, contenant une séquence d'acides aminés, comprenant : (a) dans la région N-terminale de la protéine de fusion, le fragment Fc d'une immunoglobuline ou une partie d'un fragment Fc d'une immunoglobuline qui conserve la capacité de dimérisation, comme composant (A), (b) dans la région C-terminale située plus loin de la protéine de fusion, la partie extracellulaire d'un ligand du TNF ou une séquence partielle de la partie extracellulaire d'un ligand du TNF, comme composant (B), et le cas échéant, (c) une zone de transition entre le composant (A) et le composant (B), contenant un élément de liaison,
**caractérisée en ce que** le composant (B) est la partie extracellulaire du ligand du TNF EDA1 ou est une séquence partielle de la partie extracellulaire du ligand du TNF EDA1, qui présente au moins la séquence d'acides aminés 246 à 391 de la séquence native de EDA1.

2. Protéine de fusion recombinante selon la revendication 1, **caractérisée en ce que** le composant (B) comprend la séquence d'acides aminés 140-391, 157-391, 160-391, 181-391, 182-391, 200-391 ou 245-391 de la séquence native de EDA1.

3. Protéine de fusion recombinante selon la revendication 1 ou 2, **caractérisée en ce que** le composant (A) comprend la région « charnière », le domaine CH2 et CH3 du fragment Fc d'une immunoglobuline provenant de la catégorie des IgG, en particulier de l'IgG humaine.

4. Protéine de fusion recombinante selon l'une des revendications précédentes, **caractérisée en ce que** le composant (A) présente la séquence d'acides aminés :

5. Protéine de fusion recombinante selon l'une des revendications précédentes, **caractérisée en ce que** la zone de transition entre le composant (A) et le composant (B) comprend la séquence PQPQPKPQPKPEPE.

6. Protéine de fusion recombinante selon l'une des revendications précédentes, **caractérisée en ce que** la zone de transition présente un site de clivage pour la protéase.

7. Protéine de fusion recombinante selon l'une des revendications précédentes, **caractérisée en ce que** l'extrémité N-terminale de la protéine de fusion recombinante présente une séquence signal, par exemple une séquence signal de sécrétion et/ou un marqueur tag, par exemple un tag Flag ou His.

8. Protéine de fusion recombinante selon l'une des revendications précédentes, **caractérisée en ce que** l'extrémité N-terminale présente la séquence MAIIYLILLFTAVRG.

9. Protéine de fusion recombinante selon l'une des revendications précédentes, **caractérisée en ce que** 6 composants (B) sont liés opérationnellement les uns aux autres par la séquence de la protéine de fusion recombinante.

10. Complexe constitué de protéines de fusion selon l'une des revendications 1 à 9, **caractérisé en ce que** le complexe comprend 6 chaînes du composant (A).

11. Séquence d'ADN, **caractérisée en ce que** la séquence d'ADN code pour une protéine de fusion recombinante selon l'une des revendications 1 à 9.

12. Vecteur d'expression, **caractérisé en ce que** le vecteur d'expression comprend une séquence d'ADN selon la revendication 11.

13. Cellule hôte, **caractérisée en ce que** la cellule hôte est transfectée avec un vecteur d'expression selon la revendication 12.

14. Médicament comprenant une protéine de fusion recombinante selon l'une des revendications 1 à 9, un complexe selon la revendication 10, une séquence d'ADN selon la revendication 11, un vecteur d'expression selon la revendication 12 ou une cellule hôte selon la revendication 13.

15. Utilisation d'une protéine de fusion recombinante selon l'une des revendications 1 à 9, d'un complexe selon la revendication 10, d'une séquence d'ADN selon la revendication 11, d'un vecteur d'expression selon la revendication 12 ou d'une cellule hôte selon la revendication 13 pour la préparation d'un médicament destiné au traitement des maladies génétiques, en particulier d'une dysplasie ectodermique, ou des maladies, troubles ou anomalies des structures ectodermiques, comme par exemple des anomalies du cheveux, des dents ou des glandes, en particulier pour le traitement de l'alopécie ou la cicatrisation des plaies.

16. Utilisation selon la revendication 15 pour la préparation d'un médicament destiné au traitement de la dysplasie ectodermique, en particulier de la dysplasie ectodermique hypohidrotique liée au chromosome X.

17. Utilisation selon la revendication 15 pour la préparation d'un médicament destiné au traitement de l'alopécie, de l'hirsutisme, au traitement des plaies ou d'un dysfonctionnement des glandes sudoripares ou sébacées ou d'une déficience des glandes sudoripares ou sébacées.

18. Utilisation selon l'une des revendications 15 à 17 pour la préparation d'un médicament destiné au traitement de la mère/de la femelle pendant sa grossesse/sa gravidité, dans laquelle le médicament mis à disposition est administré par voie parentérale en un dosage correspondant.
